# EUROPEAN PATENT APPLICATION

(11) **EP 2 030 624 A1**
(43) Date of publication of application: **04.03.2009**
(21) Application number: 07016789.5
(22) Date of filing: 28.08.2007
(51) Int. Cl.: A61K 31/727, A61K 31/445, A61K 45/06, A61P 39/06, A61P 7/00

(54) **Antioxidant and paramagnetic heparin-nitroxide derivatives**

(71) Applicant: Johannes Gutenberg Universität, 55122 Mainz (DE)
(72) Inventor: Kleschyov, Andrey, 55122 Mainz (DE); Golubev, Valery, Moscow region 142432 (RU); Münzel, Thomas, 55131 Mainz (DE); Sen, Vasily, Moscow region 142432 (RU)
(74) Representative: Patentanwälte Möll und Bitterich

(57) **Abstract**

The present invention relates to novel heparin-nitroxide derivatives with both antioxidant and paramagnetic properties, methods for their production, and their uses for treatment of oxidative stress-mediated diseases, for electron paramagnetic resonance imaging (EPRI), for magnetic resonance imaging (MRI), and for preservation of biological transplants. The antioxidant nitroxide derivatives of the invention comprise heparin, or a derivative thereof, and one or more nitroxides or derivatives thereof that are covalently coupled to heparin or derivative thereof by derivatization of glycosaminoglycan reactive groups.

## Description

The present invention relates to novel heparin-nitroxide derivatives with both antioxidant and paramagnetic properties, methods for their production, and their uses for treatment of oxidative stress-mediated diseases, for electron paramagnetic resonance imaging (EPRI), for magnetic resonance imaging (MRI), and for preservation of biological transplants. The antioxidant heparin-nitroxide derivatives of the invention comprise heparin, or a derivative thereof, and one or more nitroxides or derivatives thereof that are covalently coupled to heparin or derivative thereof by derivatization of glycosaminoglycan reactive groups.

Free radical formation and the effect of these toxic molecules on cell function are collectively called "oxidative stress." Oxidative stress results from an imbalance between the formation and neutralization of free radicals within cells and/or extracellular space. For instance, reactive oxygen species cause oxidative stress in endothelial cells, a condition implicated in the pathogenesis of many cardiovascular and pulmonary diseases, and diabetes.

Reactive oxygen species (ROS), such as superoxide anion radical (O₂⁻•) and H₂O₂, are formed during a variety of biochemical reactions and cellular functions. The steady-state formation of these free radicals is normally balanced by a similar rate of their consumption by antioxidants. Relatively stable O₂⁻• and H₂O₂ can generate the highly reactive ROS, such as OH• radical, thiyl radicals and peroxynitrite that can react with various cellular components including DNA, proteins, lipids/fatty acids and accelerate formation of advanced glycation end products (e.g. carbonyls). These reactions lead to the disruption of cellular compartments and/or function, e.g. DNA damage, mitochondrial malfunction, cell membrane damage and eventually cell death.

The control of ROS in the specific compartments of the cells (e.g. mitochondrium) and their concentrations is crucial for cell life. In addition, the control of the levels of free radicals in the extracellular space is of critical importance as regards to the prevention of pathological conditions such as ischemia/reperfusion, inflammation, diabetes etc. Therefore, scavenging agents have been developed both as therapeutic and diagnostic tools to neutralize damaging ROS.

The endothelium is a major site of injury caused by ROS. In one approach, scavenging of ROS at the endothelium is achieved with antioxidant enzymes, such as superoxide dismutases (SOD) and catalase (Beckman JS et al., J Free Radic Biol Med, 1986, 2(5-6):359-65). However, these enzymes are known to undergo a fast elimination from the bloodstream and permits rather modest, if any, protection against vascular oxidative stress.

It has therefore been proposed to couple polyethylene glycol (PEG) to the enzymes or encapsulate them in liposomes to increase bioavailability and to enhance the protective effect of SOD and catalase (Muzykantov VR, J Control Release, 2001, Mar 12;71(1):1-21). However, the problem still remains with this approach to scavenge free oxygen radicals at extracellular structures.

An further approach proposed chimeric protein constructs consisting of SOD and heparin-binding particles having an affinity for charged components of the endothelial glycocalix, or SOD and catalase conjugated antibodies directed against the constitutively expressed endothelial antigens, angiotensin-converting enzyme (ACE), and adhesion molecules (ICAM-1 or PECAM-1). However, none of these approaches led to a persistent and effective delivery of the antioxidants to endothelial cells or extracellular structures. Additionally, increasing SOD concentrations in tissues produce a paradoxical pro-oxidant effect (Nelson SK, Bose SK, McCord JM, Free Radic Biol Med, 1994,16:195-200), which is in part due to the SOD-derived H₂O₂ and Cu²⁺ which cause the generation of highly reactive OH• radical.

Another class of compounds that have been shown to exhibit antioxidant activity and tissue protection are nitroxides (S.M. Hahn, F.J. Sullivan, A.M. DeLuca, J.D. Becher, J. Liebmann, M.C. Krishna, D. Coffin, J.B. Mitchel, "Hemodynamic effect of the nitroxide superoxide dismutase mimics", Free Rad. Biol. Med., 1999, 27, 529-535; S. Zhang, H. Li, L. Ma, C. E. Trimble, P. Kappusamy, C.J.C. Hsia, D.L. Carden, "Polynitroxyl-albumin plus tempol attenuate lung capillary leak elicited by prolonged intestinal ischemia and reperfusion", Free Rad. Biol. Med., 2000, 29, 42-50; R. Rak, D.L. Chao, R.M. Pluta, J.B. Mitchel, E.H. Oldfield, J.C. Watson., "Neuroprotection by the stable nitroxide tempol during reperfusion in a fat model of transient focal ischemia", J. Nuerosurgery, 2000, 92, 646-651; K. Takeshita, K. Saito 1, J. Ueda, K. Anzai, T. Ozawa, "Kinetic study on ESR signal decay of nitroxyl radicals, potent redox probes for in vivo ESR spectroscopy, caused by reactive oxygen species", BBA, 2002, 1573, 156-164; T. Nassar, B. Kadery, N. Da'as, Y. Kleinman, A. Haj-Yahia, "Effects of the superoxide dismutase-mimetic compound tempol on endothelial disfunction in streptozotocin-induced diabetic rats", Eur. J. Pharmacol., 2002, 436, 111-118; J.B. Mitchel, A. Samuni, W.G. DeGraff, S. Hahn, "Nitroxides as protectors against oxidative stress", US 6605619, 8/2003; W.-J. Liaw, T.-H. Chen, Z.-Z. Lai, S.-J. Chen, A. Chen, C. Tzao, J.-Y. Wu, C.-C. Wu, "Effects of a membrane-permeable radical scavenger, tempol, on intraperitoneal sepsis-induced organ injury in rats", Shock, 2005, 23, 88-96).

Nitroxides (stable nitroxyl radicals, NR) have been used to protect cells against oxidative damage following cardiac arrest, brain, trauma, ischemia/reperfusion, and radiation (K. Patel, Y. Chen, K. Dennehy, J. Blau, S. Connors, M. Mendonca, M. Tarpey, M. Krishna, J. B. Mitchell, W. J. Welch, C. S. Wilcox, "Acute antihypertensive action of nitroxides in the spontaneously hypertensive rat", Am. J. Physiol. Regulatory Integrative Comp. Physiol., 2006, 290, 37-43; F. Hyodo, K. Matsumoto, A. Matsumoto, J. B. Mitchell, M. C. Krishna, "Probing the intracellular redox status of tumors with magnetic resonance imaging and redox-sensitive contrast agents", Cancer Research, 2006, 66, 9921-9928).

Nitroxides mimic the function of the enzyme SOD, i.e the conversion of O₂⁻• into H₂O₂ and O₂ (V.D. Sen', V.A. Golubev, I.V. Kulyk, E.G. Rozantsev, "Mechnism of the reaction of hydrogen peroxide with oxopiperidine salts and piperidinoxyl radicals", Rus. Chem. Bull.,1976, 25, 1647-1654; S. Goldstein, G. Merenyi, A. Russo, A. Samuni, "The role of oxoammonium cation in the SOD-mimic activity of cyclic nitroxides", J. Am. Chem. Soc., 2003, 125, 789-795).

The SOD-mimetic activity of nitroxides is illustrated as follows:

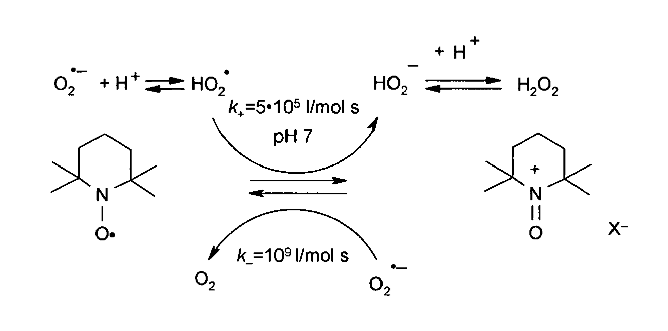

Additionally, nitroxides scavenge other ROS, such as H₂O₂, OH•, thiyl radicals etc. and undergo reversible reduction and oxidation to hydroxylamines (HAs) and oxoammonium cations (OCs) correspondingly (V.A. Golubev, Y.N. Kozlov, A.N. Petrov, A.P. Purmal, "Catalysis of redox processes by nitroxyl radicals", in "Bioactive spin labels", R.I. Zhdanov, ed., Springer, Berlin, 1992, pp. 119-140). The following scheme exemplifies the function of nitroxides in scavenging free radicals:

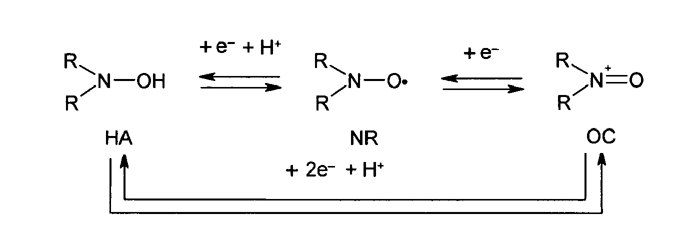

Among nitroxides, the cyclic nitroxides have been proven to exhibit unique antioxidant properties (Goldstein J. Am.Chem. Soc., 2003, (125), 789-795). Cyclic nitroxides are stablized by methyl groups at the alpha position in five-membered pyrrolidone, pyrroline or oxazolidine, and six-membered piperidine ring structures. The methyl groups confer stability to the nitroxide radicals by preventing radical-radical dismutations, and also limit access to reactive substances, which can quench the radical species.

The cyclic nitroxide TEMPO having the chemical structure of 2,2,6,6-tetramethylpiperidine-1-oxyl and its hydroxyl form, TEMPOL having the chemical structure of 4-hydroxy-2,2,6,6-tetramethylpiperidine-N-oxyl have been shown to be effective agents for potentially treating a number of oxidative stress-induced diseases. For instance, TEMPOL has been described as potential agent for treating neoplastic diseases, such as cancer (WO 2006/084197).

Since cyclic nitroxides are relatively stable free radicals, they are widely used as biophysical tools for EPR spectroscopic studies, such as spin label/oxymetry and spin trapping (McConnel HM, Spin Labeling: Theory and Applications, 1976, New York: Academic Press). As such they have been utilized as redox-sensitive paramagnetic contrast agent in Magnetic Resonance Imaging (MRI) (Matsumoto; Clin Cancer Res, 2006, 12: 2455-2462) and for EPR imaging (EPRI) (Herrling T, Free Radic Biol Med, 2003, Jul 1;35(1):59-67).

However, although nitroxides are promising agents that can be used both for tissue antioxidant defence as well as for MRI and EPRI, the problem persists that they are free flowing and are easily eliminated by rinsing solutions in vitro or by the bloodstream in vivo. As a further problem, conventional nitroxides must be used at high (mM) concentrations to be effective since they rapidly enter cells and undergo a rapid reduction process. In addition, intracellular nitroxides may negatively interfere with the cellular metabolism.

Recently, the mitochondria-targeted nitroxide (Mito-TEMPOL) has been proposed to selectively inhibit the mitochondrial oxidative damage (reviewed in: Murphy MP and Smith RAJ, Annual Review of Pharmacology and Toxicology, 2007, 47:629-656).

However, the pharmacological tools for specific targeting of nitroxides to another strategically important site, such as extracellular matrix (ECM) are still lacking. The extracellular matrix is critical for all aspects of vascular biology. In concert with supporting cells, endothelial cells assemble a laminin-rich basement membrane matrix that provides structural and organizational stability. The extracellular matrix consists of a complex mixture of proteins and glycoproteins (e.g. fibrilin) serving multiple functions such as in cellular growth development, angiogenesis and tissue regeneration. Additionally, it is known that expression and activity of the key ECM enzyme, matrix metalloproteinase(s), which is largely responsible for tumour cell metastasis and for atherosclerotic plaque instability is redox sensitive. Atherosclerosis, ischemia-reperfusion and inflammation are associated with the recruitment of activated macrophages and polynuclear leucocytes into tissue and oxidative stress in the extracellular space.
It would therefore be desirable to have a paramagnetic antioxidant nitroxide compound, which allows for selective delivery and stable association of nitroxides with cellular or extracellular structures in order to exhibit their antioxidant and paramagnetic properties.

The problem underlying the present invention is thus the provision of a therapeutically and diagnostically useful nitroxide compound with both antioxidant and paramagnetic properties which exhibits a long lasting bioavailability by binding to endothelial cell surface and/or ECM.

This problem is solved by an antioxidant heparin-nitroxide derivative according to claim 1.

The antioxidant heparin-nitroxide derivatives of the invention are able to bind to heparin binding sites on endothelial cell surface and ECM.

As used in the present invention, a derivative defines a compound that is formed from a similar compound or constitutes a modified compound. A derivative may be obtained, for instance, by replacing one atom with another atom or group of atoms. The term encompasses any modification, variant, or analogue of a compound.

A number of heparin-binding sites have been identified in the constituents of the ECM, e.g. in fibrilin and collagen, the later being a major structural protein of ECM. The antioxidant heparin-nitroxide constructs of the invention allow for directed targeting of nitroxides to the extracellular compartments. By binding to the heparin-binding sites at cell surfaces and ECM, they are able to exhibit their beneficial effects, i.e. the prevention or diminishing of oxidative stress on the cell surface and in ECM. This is particularly useful to inhibit oxidative stress in the intima layer of blood vessels. Importantly, the entry into cells and interference with intracellular metabolism is avoided.

As such the antioxidant heparin-nitroxide derivatives of the invention provide excellent therapeutic tools for treating and preventing vascular diseases such as, for instance, ischemia/reperfusion, inflammation or diabetes.

In addition to the above-mentioned therapeutic benefits, selective targeting of nitroxides to extracellular compartments potentially allows for diagnostic purposes such as EPRI and MRI of the vascular structure of vessels or monitoring of local vascular oxidative stress in vivo. Due to lack of interaction with intracellular reducing agents and prolong half life of nitroxide, heparin-nitroxides of the invention can be in particular useful for in vivo EPRI and MRI. As such, heparin being conjugated with the particular nitroxides can be used as redox, pH and dioxygen sensors in the specific tissue compartment.

Furthermore, the antioxidant heparin-nitroxide constructs of the invention can prevent oxidative stress-dependent platelet activation by scavenging of reactive oxygen species and preservation of endogenous NO activity. Thus, heparin-nitroxide of the invention can improve anticoagulant properties of such widely used drug as heparin.

The antioxidant heparin-nitroxide derivatives of the invention were produced by conjugating heparin with nitroxides.

Heparin is a member of the glycosaminoglycan family of carbohydrates (which includes the closely related molecule heparan sulfate) consisting of variably sulfated repeating disaccharide units (D.S. Milbrath, R.H. Ferber, W.E. Barnett, "Diagnostic radio-labeled polysaccharide derivatives", US 4385046, 5/1983). The main disaccharide unit of heparin is composed of 2-O-sulfo-L-idopyranosyluronic acid (1 → 4) linked to 2-N-6-O-disulfo D-glucosamine. The average heparin disaccharide contains 2.7 sulfo groups (I. Capila, R.J. Linhardt, "Heparin-protein interactions", Angew. Chem. Int. Ed., 2002, 41, 390-412; R.J. Linhardt, "Heparin: structure and activity", J. Med. Chem., 2003, 46, 2551-2564).

Heparin has the highest negative charge density of any known biological molecule and, as a result, binds efficiently to positively charged biological targets such as extracellular structures like ECM.

Native heparin is a polymer with a molecular weight ranging from 3 kDa to 50 kDa although the average molecular weight of most commercial heparin preparations is in the range of 12 kDa to 18 kDa. In its natural unfractionated state, heparin exists as a heterogeneous mixture of oligosaccharides composed of alternating chains of D-glucosamine and uronic acid.

The terms "heparin or derivative thereof" as used in the context of the present invention comprises native heparin, both fractionated and unfractioned (UH) heparin preparations, and low molecular weight heparin (LMWH). The term further encompasses any analogue or derivative of heparin such as heparan sulfate, or modified glycosaminoglycans that interact with heparin-binding sites of ECM or cell surface. Examples of such derivatives that are useful for the invention are dextran sulfates (sulfated poly-1,6-glucose), chondroitin sulfates A and C, dermantan sulfate, keratan sulfates I and II. Also encompassed by the present invention are mixtures of heparin preparations with either varying molecular weights or specific molecular weights.

Possible heparin modifications that may be used for the construction of derivatives are described, for example, in J. Gang, J. Wang, "Use of N-desulfated heparin for treating or prevention of inflammation", EP1300153, 4/2003; P.E. Thorpe, "Preparation and use of steroid-polyanionic polymer-based conjugates targeted to vascular endothelial cells", US5474765, 12/1995; P.E. Thorpe, "Method of using of steroid-polyanionic polymer-based conjugates targeted to vascular endothelial cells", US5762918, 6/1998; Y. Byun, Y.-K. Lee, "Oral delivery of macromolecules", US2002/0010153, 1/2002; A.D. Cardin, C.L. Van Gorp, "Targeted agents useful for diagnostic and therapeutic applications", US6409987, 6/2002.

Preferably the heparin or derivative thereof as utilized in the antioxidant heparin-nitroxide derivative of the invention has a molecular weight of approximately between 5 and 40 kDa. Most preferred is around 15 KDa of fractionated heparin.

The following schemes illustrate possible ways of a conjugation of heparin with nitroxide (R) via an optional linker (L). By adding a linker chain L, the nitroxide molecule will be placed in greater distance from the heparin macromolecule, which results in a higher spectral mobility as further shown in the Examples. The basic structure of the heparin macromolecule is shown in scheme 1 a, possible heparin-nitroxides conjugations are shown in schemes 1 b and 1c, respectively.

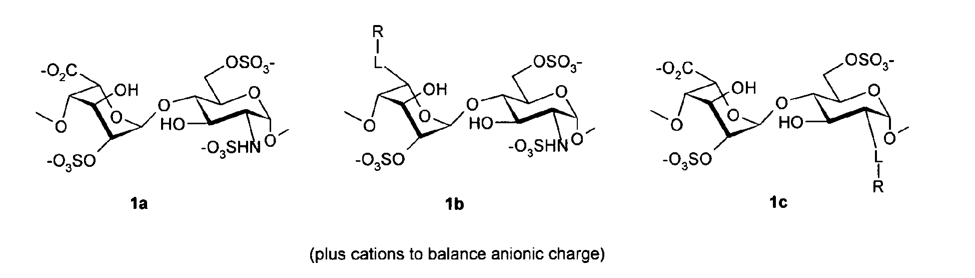

Preferred nitroxides or their derivatives according to the invention are derived from cyclic nitroxides. A preferred nitroxide to be utilized in the present invention is TEMPO (2,2,6,6-tetramethylpiperidine-1-oxyl). Preferred nitroxides or derivatives thereof are derived from piperidine, tetrahydropyridine, pyrroline, pyrrolidine, imidazoline, imidazolidine or oxazolidine.

### Methods of making antioxidants heparin-nitroxide constructs of the invention

Nitroxide or its derivative can be linked to the glycosaminoglycan backbone of heparin by derivatization of glycosaminoglycan reactive groups. Preferred reactive groups for coupling nitroxide to heparin are COOH, NH₂ or OH in their oxidised or non-oxidised forms.

Although any of known coupling reactions may be used for making the heparin-nitroxide derivatives of the invention, there are two preferred ways of derivatization glycosaminoglycan reactive groups of heparin:
1. Derivatization of heparin carboxyl groups with amino group containing nitroxides, or
2. Derivatization of heparin amino groups with carboxyl group containing nitroxides. Any of the -COOH, -NH₂ (after desulfation) and -OH groups of the heparin macromolecule are suitable for derivatization with nitroxides. However, poor solubility of heparin in solvents other then water or water-DMF (DMSO) mixtures may eventually limit the options for reagents options and types of activation reaction for derivatization. Soluble non-polar organic solvents heparin salts associated with bulky ammonium cations are preferred when the derivatization reagents, such as acid chlorides, require anhydrous reaction conditions.

### 1. Derivatization of heparin carboxyl groups

One preferred method of making the heparin-nitroxide derivatives of the invention comprises carbodiimide-mediated coupling of heparin carboxyl groups with amino group containing substances of interest (see formula 1b, above). This reaction results in heparin derivatives having the following general formula, wherein the nitroxide R is a 5- or 6-atom N-heterocycle, L is a linker which comprises amide -C(O)NR'-, ester -C(O)O- or their combination separated by hydrocarbon chain -C(O)NR'-(CH₂)ₙ-C(O)O- or by any other suitable linker, R' is a hydrogen or an alkyl substituent, n indicates the chain length of the linker, wherein n ≥ 0, preferably n is an integer >1, and x,y > 0.

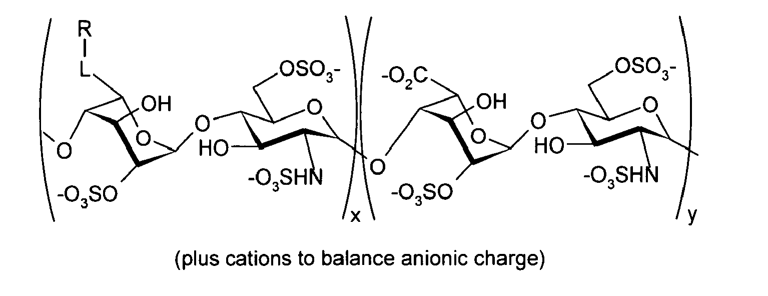

Any amino group containing nitroxide, including nitroxides with an additional heteroatom in the backbone cycle, like imidazoline, imidazolidine or oxazolidine, are suitable for the derivatization of heparin according to the method of the invention.

Preferred nitroxides of the present invention are those with the highest k+ limiting rate constant, i.e. nitroxides that are predominantely oxidizable by HO₂• radicals (HO₂• ↔ O₂•- + H⁺).

Piperidine nitroxides are preferable as SOD-mimetics because the pyrrolidine/pyrroline nitroxide reaction with HO₂• is characterized by a lower k+ limiting rate constant.

Imidazoline and imidazolidine types of nitroxides bound to heparin are preferable as extracellular pH-sensitive EPR probe.

Per-deuterated nitroxides which are known to exhibit a very narrow EPR signal (0.08 gauss) are preferred for EPRI purpose and as oxymetry probe.

A preferred solvent for heparin is water. In a further embodiment, water-soluble carbodiimides are preferable such as, for instance, N-(3-dimethylaminopropyl) N'-ethylcarbodiimide hydrochloride (EDC).

Furthermore, the use of auxiliary N-hydroxy succinimide (NHS) can improve the efficiency of amide bond formation:

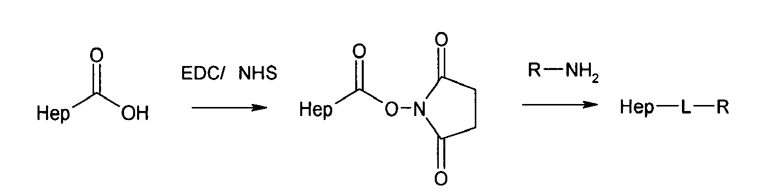

In this scheme, Hep-COOH refers to the heparin macromolecule with one of its carboxyl group, R-NH₂ refers to an amino group containing nitroxide radical and L is a linker. Any suitable linker may be used for the invention that is able to conjugate nitroxide to the heparin macromolecule. The length of the linker may influence the antioxidant and paramagnetic properties of the bound nitroxyl radical. A longer linker chain, for instance, places the nitroxide molecule in greater distance from the heparin backbone, and thus may give different EPR signal properties. In fact, as shown in the Examples below, the signal width of the EPR spectrum (X- or L-band) may be influenced by the linker length. A preferred linker as used in the present invention is characterized by the formula - (CH₂)ₘ-, wherein m is an integer > 0. Preferably m is an integer from 1 to 6. However, as it will be apparent for the person skilled in the art, any suitable linker known in the art can be used for the purposes of the present invention.

For example, a non-substituted amide bond -C(O)NH- may be used as a simple form of a linker. Additional possible linker L structures are presented under the below. Variations in L structure can be obtained by combining nitroxides such as, for instance, HO(CH₂)n-, HOOC(CH₂)n- or H2N(CH₂)n- (n = 0-2) with linear bifunctional agents such as, for instance, H₂N(CH₂)mNH₂, H₂N(CH₂)mOH, H₂N(CH₂)mCOOH, natural amino acids or short peptides in analogous manner. Preferably, the m values are within the range from 1 to 6. Linkers with cyclic bifunctional moieties may also be used in the same manner.

Amino group bound nitroxides with flexible linkers of variable length may be obtained as shown for 4-[(5-aminopentyl)carbonylamino]-2,2,6,6-tetramethylpiperidine-1-oxyl (see also Example 1):

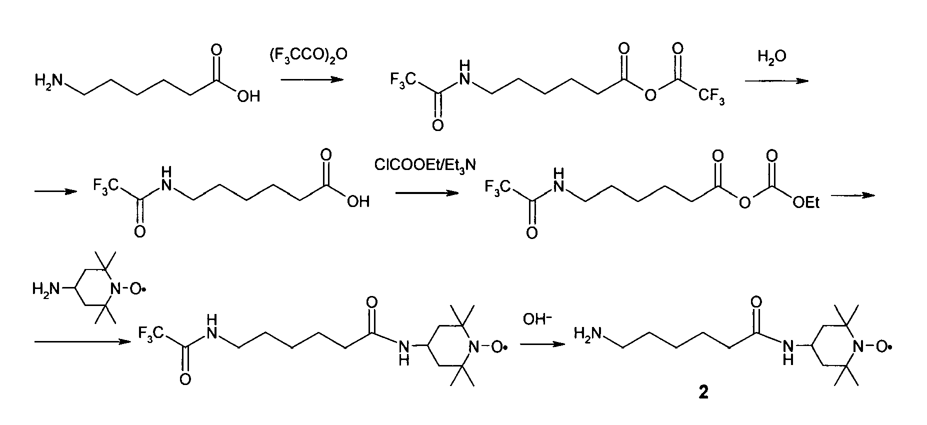

For the coupling reaction, the preferred NHS/R-NH₂ molar ratios are within the range from 1:10 to 1:1. In order to obtain higher degrees of derivatization, carbodiimide is preferably added to the mixture of heparin/NHS/amino-nitroxide rather than the other way round. The degree of heparin derivatization depends substantially on the temperature profile of the reaction. The best results are obtained when during the first 30 to 90 min the reaction mixture is kept in an ice bath and is then subsequently warmed up to a temperature of around 20°C. Depending on the relationship of the reagents and the duration of the reaction, the degree of heparin derivatization may be changed up to one radical per disaccharide unit.

Preferred nitroxides as used in the present invention comprise HO(CH₂)n-, HOOC(CH₂)n- or R'HN(CH₂)n-functionalyzed nitroxides, wherein n is an integer from 0 to 2, and R' is H or Me.

In preferred antioxidant heparin-nitroxides derivatives, the following nitroxide radical moieties are suitable:
R1 = 2,2,6,6-tetramethyl-1-oxylpiperidin-4-yl,
R2 = 3-amino-2,2,6,6-tetramethyl-1-oxylpiperidin-4-yl,
R3 = 4-alkyloxycarbonyl-2,2,6,6-tetramethyl-1-oxylpiperidin-4-yl,
R4 = 4-hydroxyimino-2,2,6,6-tetramethyl-1-oxylpiperidin-3-yl,
R5 = 2,2,5,5-tetramethyl-1-oxylpyrrolidin-3-yl,
R6 = 2,2,6,6-tetramethyl-1-oxylpiperidin-4-diyl,
R7 = 2,2,5,5-tetramethyl-1-oxylpyrrolidin-3-diyl,
R8 = 2,2,6,6-tetramethyl-1-oxyl-1,2,5,6-tetrahydropyridin-4-yl,
R9 = 4-acetylamino-2,2,6,6-tetramethyl-1-oxylpiperidin-4-yl,
R10 = 2,2,5,5-tetramethyl-1-oxylpyrrolin-3-yl,
R11 = 2,2,5,5-tetramethyl-4-bromo-1-oxylpyrrolin-3-yl,
R12 = 2,2,6,6-tetramethyl-1-oxyl-4-phenylpiperidin-4-yl.

In the following, structures of antioxidant heparin-nitroxides derivatives of preferred embodiments are presented as obtained by coupling amino group containing nitroxide to carboxyl group containing heparin. For further details on their production please refer to the Examples.

One amide bond linkers (see Examples 2, 3):

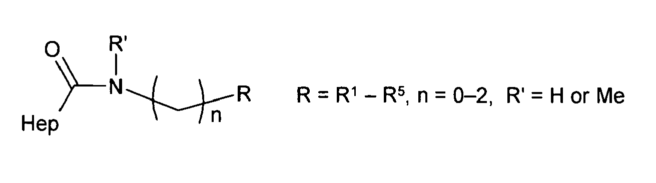

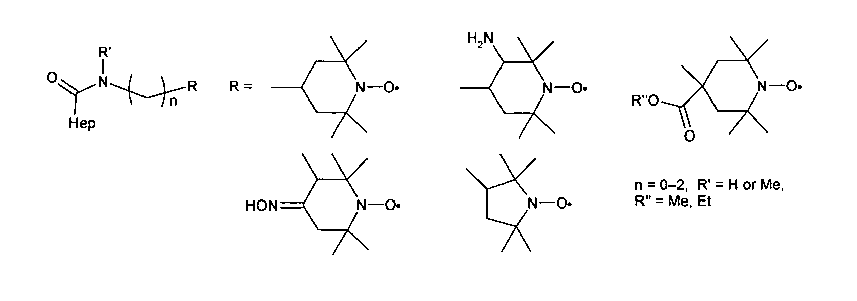

Hydrazide-hydrazone bond linker (see Examples 5-7):

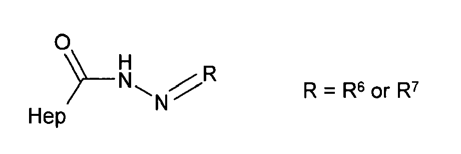

Two amide bonds linkers (see Example 4): 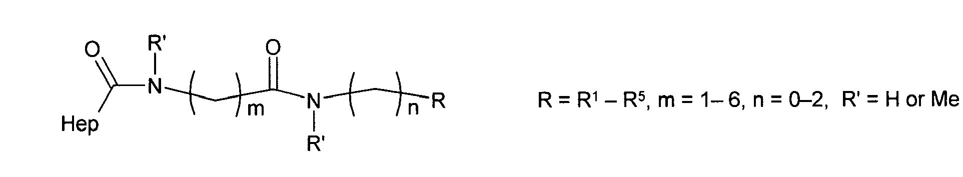 or

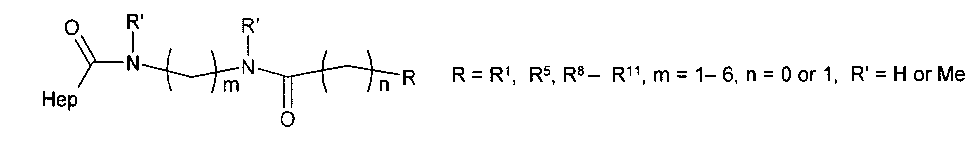

Amide and ester bond linkers: 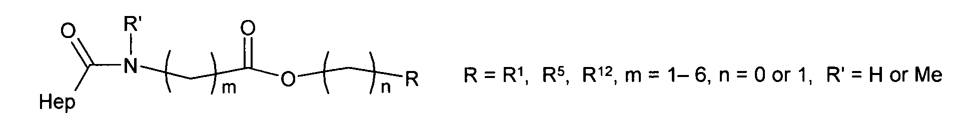 or 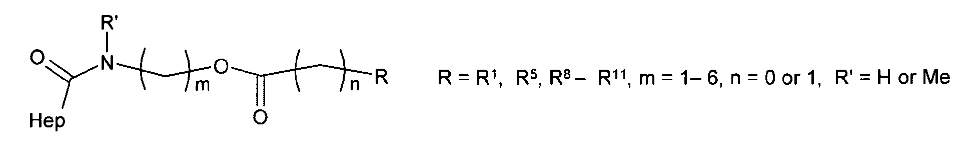 wherein R = R¹⁻¹² may be one of the following residues:
R¹ = 2,2,6,6-tetramethyl-1-oxylpiperidin-4-yl,
R² = 3-amino-2,2,6,6-tetramethyl-1-oxylpiperidin-4-yl,
R³ = 4-alkyloxycarbonyl-2,2,6,6-tetramethyl-1-oxylpiperidin-4-yl,
R⁴ = 4-hydroxyimino-2,2,6,6-tetramethyl-1-oxylpiperidin-3-yl,
R⁵ = 2,2,5,5-tetramethyl-1-oxylpyrrolidin-3-yl,
R⁶ = 2,2,6,6-tetramethyl-1-oxylpiperidin-4-diyl,
R⁷ = 2,2,5,5-tetramethyl-1-oxylpyrrolidin-3-diyl,
R⁸ = 2,2,6,6-tetramethyl-1-oxyl-1,2,5,6-tetrahydropyridin-4-yl,
R⁹ = 4-acetylamino-2,2,6,6-tetramethyl-1-oxylpiperidin-4-yl,
R¹⁰ = 2,2,5,5-tetramethyl-1-oxylpyrrolin-3-yl,
R¹¹ = 2,2,5,5-tetramethyl-4-bromo-1-oxylpyrrolin-3-yl,
R¹² = 2,2,6,6-tetramethyl-1-oxyl-4-phenylpiperidin-4-yl.

The number of molecules of nitroxides over the full length of the heparin macromolecule can vary. A preferred number of nitroxides per heparin macromolecule is, for instance, approximately 2 to 24 nitroxides per heparin macromolecule having a molecular weight of around 15 kDa. In a preferred embodiment, about 20 % to 70 % of the disaccharides of heparin are modified by the cyclic nitroxide TEMPO. In further embodiments, around 20 %, 45 % or 70 % of the disaccharides of heparin are modified by TEMPO. In a yet preferred embodiment more than 70% of the disaccharides of heparin are modified by TEMPO.

### 2. Heparin amino groups derivatization

The second preferred method of making the antioxidant heparin-nitroxides of the invention comprises N-desulfation/N-acylation of heparin resulting in heparin-nitroxide derivatives of the following general formula: 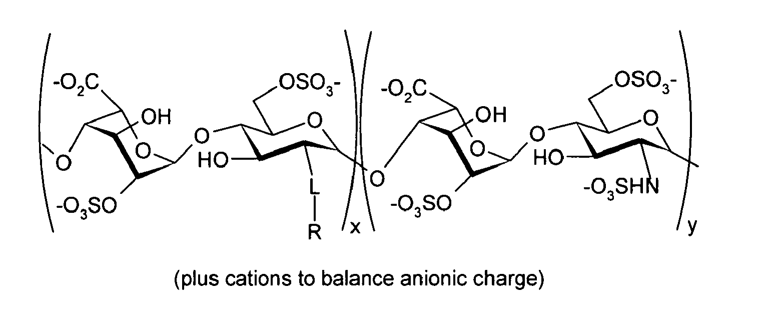 wherein the nitroxide R is a 5- or 6-atom N-heterocycle, L is a linker which comprises amide -C(O)NR'-, ester -C(O)O- or their combination separated by hydrocarbon chain -C(O)NR'-(CH₂)ₙ-C(O)O- or by any other suitable linker, R' is a hydrogen or an alkyl substituent, n indicates the chain length of the linker, wherein n ≥ 0, preferably n is an integer >1, and x,y > 0.

The derivatization of heparin amino groups is preferably performed after N-desulfation of heparin. In a preferred method of the invention, NHS esters of carboxyl group containing nitroxides (NHS-C(O)R) were obtained and coupled to -NH₂ groups of N-desulfated heparin Hep-NH₂ in a water-DMSO mixture according to the following reaction scheme:

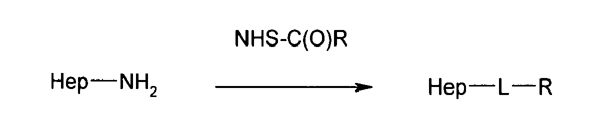

In this scheme, L represents a linker as defined under the above. In principle, any carboxyl group containing nitroxide is suitable for heparin derivatization according to this variant of the method of the invention.

In the following, structures of antioxidant heparin-nitroxides derivatives are presented as obtained by coupling carboxyl group containing nitroxides or derivative thereof to amino group containing heparin or a derivative thereof. For further details as regards to their production please refer to the Examples that follow under the below.

One amide bond linkers (see Example 8):

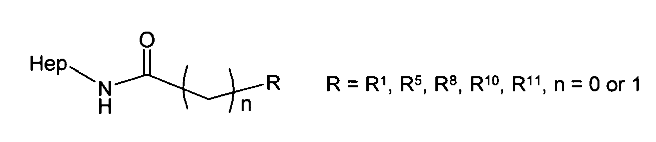

Two amide bond linkers: 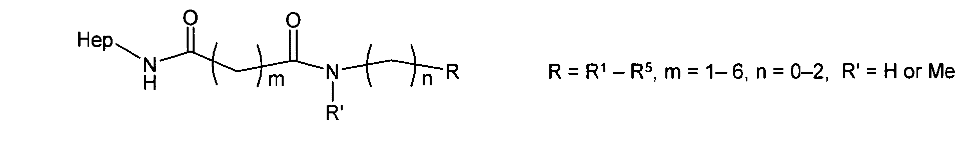 or

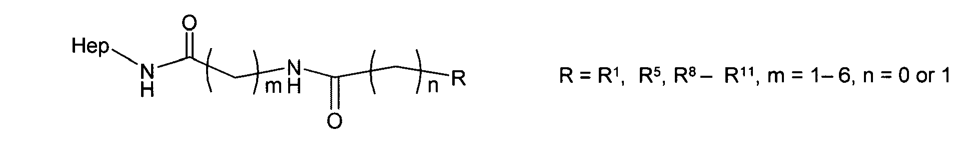

Amide and ester bond linkers: 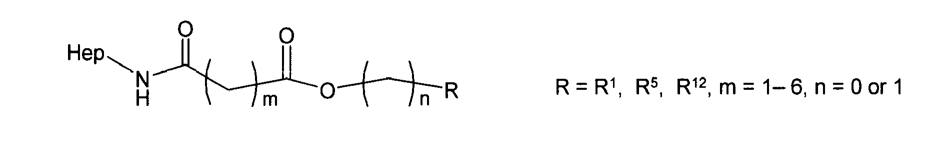 wherein R = R¹⁻¹² may be one of the following residues:
R¹ = 2,2,6,6-tetramethyl-1-oxylpiperidin-4-yl,
R² = 3-amino-2,2,6,6-tetramethyl-1-oxylpiperidin-4-yl,
R³ = 4-alkyloxycarbonyl-2,2,6,6-tetramethyl-1-oxylpiperidin-4-yl,
R⁴ = 4-hydroxyimino-2,2,6,6-tetramethyl-1-oxylpiperidin-3-yl,
R⁵ = 2,2,5,5-tetramethyl-1-oxylpyrrolidin-3-yl,
R⁶ = 2,2,6,6-tetramethyl-1-oxylpiperidin-4-diyl,
R⁷ = 2,2,5,5-tetramethyl-1-oxylpyrrolidin-3-diyl,
R⁸ = 2,2,6,6-tetramethyl-1-oxyl-1,2,5,6-tetrahydropyridin-4-yl,
R⁹ = 4-acetylamino-2,2,6,6-tetramethyl-1-oxylpiperidin-4-yl,
R¹⁰ = 2,2,5,5-tetramethyl-1-oxylpyrrolin-3-yl,
R¹¹ = 2,2,5,5-tetramethyl-4-bromo-1-oxylpyrrolin-3-yl,
R¹² = 2,2,6,6-tetramethyl-1-oxyl-4-phenylpiperidin-4-yl.

### 3. Alternative methods for making antioxidant heparin-nitroxide derivatives

Another way for making the antioxidant heparin-nitroxide derivatives of the invention relates to heparin -OH and/or -NH2 groups derivatization under anhydrous conditions.

Nitroxide radicals with carboxylic acid anhydride or acid chloride functional groups are suitable for -OH and/or -NH2 groups acylation of heparin salts with bulky ammonium cations in polar organic solvents (for details of analogous reactions see M. Petitou, C. Coudert, M. Level, J.-C. Lormeau, M. Zuber, C. Simenel, J.-P. Fournier, J. Choay, "Selectively O-acetylated glycosaminoglycan derivatives", Carbohydr. Res., 1992, 236, 107-119). Isocyanato nitroxides can also be used for coupling of nitroxides to heparin through -OH and/or -NH2 groups under anhydrous conditions (W. Marconi, F. Benvenuti, A. Piozzi, "Covalent bonding of heparin to a vinyl copolymer for biomedical applications", Biomaterials, 1997, 18, 885-890).

### Synthesis and characterization of heparin-nitroxides

The progress of the reactions was monitored by HPLC, TLC, and EPR techniques. A Milikhrom chromatograph was used for HPLC (column 2x64 mm, Separon C18 (5 µm), detection at 240 nm) with the use of 30% aqueous MeCN containing KH2PO4 (0.05 M) as the eluent. TLC was carried out on Silufol UV254 0.25-mm silica gel plates. Visualization of the TLC plates was by one of the following methods: (1) UV light (254 nm), or (2) staining solution (0.3% ninhydrin in water containing 3% acetic acid) followed by heating. IR spectra were recorded in the range of 400 to 4000 cm⁻¹ on a Specord 75-IR spectrometer in Nujol. EPR spectra were measured at room temperature on an SE/X 2544 instrument at a UHF power of 2 mW and a modulation of 0.032 mT. Mass spectrum was recorded on a Finnigan-4021 (IE, 55 eV) apparatus.

For quantitative analysis, the samples of reaction mixtures were taken and the unbounded NRs were determined by HPLC and/or EPR after precipitation of the polymer by excess of MeCN. For heparin carboxyl group derivatization, the fraction of modified heparin disaccharides was calculated as α = 615m/a, were 615 is the assumed average MW of disaccharide unit in unmodified heparin, m is the quantity (mM) of NR bonded to polymer (found as the difference between the quantities of the NR introduced into the reaction and the unbounded NR in the mother liquid after precipitation of the modified polymer at the end of the reaction) and a is the quantity of heparin taken for the reaction (mg).

For heparin N-desulfation and subsequent complete NH₂-group acylation, the fraction of modified heparin disaccharides was calculated as α = 615m/(a + 102m), were 102 is the difference in MW of unmodified and 100% N-desulfated heparin (-SO₃Na + H). The degree of heparin derivatization was found also by double integration of the ESR spectra of the modified heparin (2 - 3 mg/ml water solutions) in comparison to the known concentration (5x10⁻⁴ M in water) of the reference radical 2,2,6,6-tetramethylpiperidine-1-oxyl. By comparison of the both methods of α value determinations, it was found that the integration method undervalue it by 0 to 20 relative %, presumably, due to the double integration error of the broadened spectra (the greater was the broadening, the higher was the deviation).

Heparin (H4784) was purchased from Sigma, trifluoroacetic anhydride, ethyl chloroformate, N-hydroxy succinimide and N-(3-dimethylaminopropyl) N'-ethylcarbodiimide hydrochloride were purchased from Aldrich and utilized as received. Nitroxide radicals 4-amino-2,2,6,6-tetramethylpiperidine-1-oxyl, 3-amino-2,2,5,5-tetramethylpyrrolidine-1-oxyl, 4-oxo-2,2,6,6-tetramethylpiperidine-1-oxyl, 3-oxo-2,2,5,5-tetramethylpyrrolidine-1-oxyl and 2,2,6,6-tetramethylpiperidine-1-oxyl were synthesized according to the described methods (Rozantsev EG, "Free nitroxyl radicals", Plenum Pres, New York, 1970), 2,2,6,6-tetramethyl-4-(succinimidooxycarbonylmethyl)piperidine-1-oxyl was made according to (Maksimova LA, Grigoryan GL, Rozantsev EG, Rus. Chem. Bull., 1975, 24: 859-862) and 4-hydrazono-2,2,6,6-tetramethylpiperidine-1-oxyl according to (H. Schlude, "A new reagent for the spin labeling of aldehydes and ketones", Tetrahedron Lett., 1976, 25, 2179-2182). Solvents were purified by standard procedures and distilled.

The structure of the antioxidant heparin-nitroxide derivatives of the invention is in agreement with their EPR- and IR-spectra (see Figures). Antioxidant nitroxide-heparin derivatives with the general formula Hep-L-NR are characterized by three-line EPR-spectra, which arise from the splitting of an unpaired electron signal on the 14N-nucleus.

The degree of broadening of the lines in the EPR-spectra depends on the nature of the linker L. Nitroxyl radicals coupled to the macromolecule with long flexible linkers have greater mobility and exhibit more narrow lines spectra. Compared to unmodified heparin, the IR-spectra of nitroxide heparin derivatives exhibit a new band at ∼1550 cm⁻¹ (-CO-NH-, amide II band), which is indicative of amide bond formation according to the reaction scheme above. The amide I band due to the C=O stretching vibrations overlaps with the broad band of the heparin -CO₂-groups (1625 cm⁻¹ for unmodified heparin) and the maximum of the resulting band is at 1640-1655 cm⁻¹ (see Examples). The other expected band (C=O stretching vibrations, amide I band) overlaps with the broad band of the heparin -CO₂-groups (1625 cm⁻¹ for unmodified heparin) and the maximum of the resulting band is at 1640-1655 cm⁻¹.

### Examples

### Example 1

Synthesis of 4-[(5-aminopentyl)carbonylamino]-2,2,6,6-tetramethylpiperidine-1-oxyl. Mixture of 1.31 g (10 mM) of 6-aminocaproic acid and 4.5 ml of trifluoroacetic anhydride was heated for 2 h at 80°C in soldered ampoule. Volatile components of the reaction mixture were evaporated at reduced pressure. The residual liquid consisted mainly of bis-trifluoroacetylated 6-aminocaproic acid. To achieve hydrolysis of mixed anhydride function of this intermediate, 0.25 ml (14 mM) of water was added to it with ice cooling. The solution was left for 1 h at ∼20°C and after that it was azeotroped with three 10 ml portions of dry benzene. The yield of 6-(trifluoroacetylamino)caproic acid was 2.27 g, mp 83°C. It was dissolved in 10 ml of ethyl acetate and triethylamine (1.39 ml, 10 mM) and ethyl chloroformate (0.96 ml, 10 mM) were added sequentially at ice bath cooling and stirring. After stirring for 20 min at the same cooling, solution of 4-amino-2,2,6,6-tetramethylpiperidine-1-oxyl (1.71 g, 10 mM) in 2.5 ml of ethyl acetate was added in 5 min time interval. The mixture was allowed to warm to room temperature and stirred for additional 30 min. The triethylammonium chloride salt was filtered off and washed with ethyl acetate (3ml x 3). Red ethyl acetate solution was washed sequentially with 2 ml of 0.1 M HCl, 1 ml of water and 2 ml of saturated NaHC03 solution and dried over anhydrous MgSO4. Ethyl acetate was removed in vacuo to yield 3.9 g of 4-[(5-trifluoroacetylaminopentyl)carbonylamino]-2,2,6,6-tetramethylpiperidine-1-oxyl as a red oil. It was dissolved in 7 ml of ethanol and 11 ml of 1 M NaOH solution in water was added at ∼10°C. The mixture was left at room temperature for 20 h. Solid K2CO3 (10 g) and 10 ml of ethyl acetate were added with stirring. The upper organic layer was separated, washed with saturated aqueous solution of NaCl, dried over anhydrous MgSO₄ and the solvent was removed in vacuo. The product was purified by column chromatography (silica gel, 5:1 to 2:1 chloroform/methanol) yielding 2.3 g of 4-[(5-aminopentyl)carbonylamino]-2,2,6,6-tetramethylpiperidine-1-oxyl as red hygroscopic solid, which did not have an sharp melting point. Found (%): C, 62.95; H, 10.55; N, 14.35. C15H30N3O2. Calculated (%): C, 63.34; H, 10.63; N, 14.77. MW 284.421. IR (Nujol mull), v/cm-1: 1543 (O=CNH), 1645 (C=O), 3265 (OCN-H), 3335, 3445 (NH2). MS (55 eV), m/z (I, %): 285 [M+1]+(4.5), 284 [M]+(0.6), 198 (24), 155 (2.5), 140 (16), 124 (13), 114 (11), 109 (11), 98 (11), 84 (100), 70 (38), 55 (43), 44 (49), 43 (65). EPR (H2O, 5•10-4 M): three lines, g factor was 2.0056, aN = 1.70 mT.

### Example 2

Coupling of amino nitroxide to the heparin carboxyl groups. 171 mg (1 mM) of 4-amino-2,2,6,6-tetramethylpiperidine-1-oxyl (H2N-R1) and 115 mg of N-hydroxy succinimide (NHS) were mixed with solution of 615 mg of heparin sodium salt in 10 ml of 0.1 M HCl. The solution obtained was cooled in an ice bath and N-(3-dimethylaminopropyl) N'-ethylcarbodiimide hydrochloride (EDC) (230 mg, 1.2 mM) was added with stirring. The resulting solution was stirred in the ice bath for 30 min and after that stirring was continued at ∼20°C. H2N-R1 consuming was monitored by HPLC. Optimal pH∼5 was maintained by adding of 0.1 M NaOH solution. After 7 h 120 mg (0.70 mM) of H2N-R1 was coupled to heparin. The reaction mixture was freeze dried until it weight was ∼4 g and absolute ethanol (30 ml) was added slowly with stirring. The precipitate was triturated to powder, filtered, washed with absolute ethanol (3 ml x 3), dissolved in water (3 ml) and reprecipitated with absolute ethanol (30 ml). The precipitate was washed with absolute ethanol and vacuum dried, yielding 660 mg of Hep-C(O)NH-R1 as a pale pink powder. The nitroxide radical content in the product was found to be 1.14 x 10⁻³ M/g (mol of R1 per gram of the derivate). For disaccharide unit assumed MW = 615, it meant 70% of heparin carboxyl groups derivatization. IR of Hep-C(O)NH-R1 (Nujol mull), v/cm-1: 1000, 1030, 1235 (SO3-), 1550(O=CNH), 1655 (O=CNH + CO2-). EPR (H2O, 3 mg/ml): three lines with 100:115:60 heights ratio, g factor was 2.0056, aN = 1.70 mT.

### Example 3

Using the method of Example 2, 3-amino-2,2,5,5-tetramethylpyrrolidine-1-oxyl (H2N-R5) was coupled to heparin, time of reaction was 4.5 h. The nitroxide radical content in the product was found to be 1.31 x 10⁻³ M/g or 81% of heparin carboxyl groups derivatization. IR of Hep-C(O)NH- R5 (Nujol mull), v/cm-1: 1000, 1030, 1235 (SO3-), 1553(O=CNH), 1657 (O=CNH + CO2-). EPR (H2O, 3 mg/ml): three lines with 100:130:50 heights ratio, g factor was 2.0053, aN = 1.58 mT.

### Example 4

Using the method of Example 2, 4-[(5-aminopentyl)carbonylamino]-2,2,6,6-tetramethylpiperidine-1-oxyl [H2N(CH2)5C(O)NH-R1] was coupled to heparin, time of reaction was 24 h. The nitroxide radical content in the product was found to be 7.5 x 10-4 M/g. For disaccharide unit estimated MW = 615, it meant 46% of heparin carboxyl groups derivatization. IR of Hep-C(O)HN(CH2)5C(O)NH-R1 (Nujol mull), v/cm-1: 1000, 1030, 1235 (SO3-), 1548(O=CNH), 1645 (O=CNH + CO2-). EPR (H2O, 2.7 mg/ml): three lines with 100:101:68 heights ratio, g factor was 2.0056, aN = 1.70 mT.

### Example 5

Using the method of Example 2, 4-hydrazono-2,2,6,6-tetramethylpiperidine-1-oxyl (H2NN=R6) was coupled to heparin, time of reaction was 5 h. The nitroxide radical content in the product was found to be 1.18 x 10⁻³ M/g. For disaccharide unit estimated MW = 615, it meant 73% of heparin carboxyl groups derivatization. IR of Hep-C(O)NHN=R6 (Nujol mull), v/cm-1: 1000, 1030, 1235 (SO3-), 1570(O=CNH), 1655 (O=CNH + CO2-). EPR (H2O, 5 mg/ml): three lines with 100:140:60 heights ratio, g factor was 2.0056, aN = 1.70 mT.

### Example 6

Heparin sodium salt (62 mg) was dissolved in 1 ml of 0.1 M hydrazine monohydrochloride in water. To this solution NHS (16 mg, 0.15 mM) and EDC (20 mg, 0.1 mM) were added sequentially and the mixture was left for 20 h at ∼20°C. The reaction mixture was freeze dried until it weight was ∼0.25 g and absolute ethanol (4 ml) was added slowly with stirring. The precipitate was triturated to powder, filtered, washed with absolute ethanol (2 ml x 2), and vacuum dried, yielding 66 mg of Hep-C(O)NHNH2. It was dissolved in 0.5 ml of water, 4-oxo-2,2,6,6-tetramethylpiperidine-1-oxyl (O=R6) (43 mg, 0.25 mM) was added to the solution and the mixture was kept at ∼20°C. O=R6 consuming was monitored by HPLC. After 20 h 14 mg (0.082 mM) of H2N-R1 was coupled to heparin. The reaction mixture was freeze dried until it weight was ∼0.2 g and absolute ethanol (4 ml) was added slowly with stirring. The precipitate obtained was washed with absolute ethanol (1 ml x 2) and vacuum dried, yielding 53 mg of Hep- Hep-C(O)NHN=R₆ as a pale yellow powder. The nitroxide radical content in the product was found to be 1.34 x 10⁻³ M/g and meant 70% of heparin carboxyl groups derivatization. The IR and EPR characteristics of the derivate were the same as in example 5.

### Example 7

Using the method of Example 6, 3-oxo-2,2,5,5-tetramethylpyrrolidine-1-oxyl (O=R7) was coupled to heparin, time of reaction was 20 h. The nitroxide radical content in the product was found to be 1.46 x 10⁻³ M/g and meant 90% of heparin carboxyl groups derivatization. IR of Hep-C(O)NHN=R7 (Nujol mull), v/cm-1: 1000, 1030, 1235 (S03-), 1575(O=CNH), 1655 (O=CNH + CO2-). EPR (H2O, 5 mg/ml): three lines with 100:137:62 heights ratio, g factor was 2.0053, aN = 1. 57 mT.

### Example 8

N-desulfation of heparin was performed by modification of described methods (D.S. Milbrath, R.H. Ferber, W.E. Barnett, "Diagnostic radio-labeled polysaccharide derivatives", US 4385046, 5/1983; A. I. Usov, K. S. Adamyants, L. I. Miroshnikova, A. A. Shaposhnikova, N. K. Kochetkov, "Solvolytic desulphation of sulphated carbohydrates", Carbohydr. Res., 1971, 18, 336-338; Y. Inoue, K. Nagasawa, "Selective N-desulfation of heparin with dimethyl sulfoxide containing water or methanol", Carbohydr. Res., 1976, 46, 87-95; L. Huang, R.J. Kerns, "Diversity-oriented chemical modification of heparin: Identification of charge-reduced N-acyl heparin derivatives having increased selectivity for heparin-binding proteins", Bioorg. Med. Chem., 2006, 14, 2300-2313). Sodium heparin (280 mg) was converted to the pyridinium salt by dissolving in water and passing through cationic exchange resin (Dowex 50WX4-400, H+ form) at the ice bath cooling. Double excess of pyridine was added relative to the total number of the acidic groups in heparin (the final pH ∼5). The solution obtained was frozen and lyophilized. The pyridinium heparin obtained (288 mg) was dissolved in 10 ml DMSO/water (95:5) and stirred in 35°C water bath for 70 min. lce-cooled water (10 ml) was added and the pH was adjusted to 9 with 0.2 N NaOH at ice-cooling. The solution was dialyzed against water (MWCO = 2000) and rotary evaporated at 20°C water bath to residual weight of ∼0.7 g. Absolute alcohol (5 ml) was added with mixing. The precipitate was filtered, washed with absolute ethanol (3 ml x 2) and vacuum dried, yielding 203 mg of partially N-desulfated heparin Hep-NH₂. It was dissolved in 10 ml of DMSO/H₂O (2:1), saturated with NaHCO₃. 94 mg (0.30 mM) of 2,2,6,6-tetramethyl-4-(succinimidooxycarbonylmethyl)piperidine-1-oxyl (crystallized from ethanol, mp 172-174°C) was pounded with a pestle and added to Hep-NH2 solution with stirring (∼ 1 equiv of NHS ester per 1 equiv disaccharide unit). Mixing was continued at ∼20°C.

By HPLC and EPR analysis of the reaction mixture after 12 and 24 h it was found that 1) 0.23 (12 h) and 0.24 (24 h) mM of NHS ester was coupled to heparin; 2) some quantity of the starting NHS ester still existed in the mixture after 24 h reaction time; 3) pH of the reaction mixture was in the range 8.5 - 9. From this data it was concluded that all free amino groups of Hep-NH2 were acylated.

The volume of reaction mixture was halved by freeze drying and the polymer was precipitated by addition of excess of acetone/ether (1:1), centrifuged, decanted and washed with acetone. Dried polymer was dissolved in water (1.5 ml), centrifuged, decanted and re-precipitated with absolute ethanol (15 ml). The precipitate was washed with absolute ethanol and vacuum dried, yielding 189 mg of Hep-NHC(O)CH2-R1 as a pale orange powder. The fraction of modified disaccharides in the product α was found to be 0.65. It meant 65% disaccharide modification and the nitroxide radical content in the product equal to 9.6•10-4 M/g. IR of Hep-NHC(O)CH2-R1 (Nujol mull), v/cm-1: 985, 1020, 1240 (SO3-), 1535(NHC=O), 1640 (NHC=O + CO2-). EPR (H2O, 2.1 mg/ml): three lines with 100:127:20 heights ratio, g factor was 2.0055, aN = 1.71 mT.

### Biological and paramagnetic properties

As such the antioxidant nitroxide heparin derivatives of the invention are excellent tools for both therapeutic applications and EPR imaging. The antioxidant nitroxide heparin derivatives of the invention describe for the first time an extracellular-superoxide-dismutase mimetic.

The invention also encompasses a pharmaceutical composition, comprising an antioxidant heparin-nitroxide derivative of the invention. The pharmaceutical composition may contain any suitable carrier, solvent, vehicle or excipient for stable storage and efficient delivery of the therapeutic agent to its target.

The antioxidant heparin-nitroxide derivative of the invention can be used as therapeutic agent for the preparation of a medicament for treatment of acute and chronic diseases that are associated with oxidative extracellular stress.

In particular, the disease associated with oxidative extracellular stress to be treated by the antioxidant heparin-nitroxide derivative of the invention is selected from the group consisting of oxidative stress-dependent platelet activation, cardiovascular disease, neurodegenerative diseases such as Alzheimer's, pulmonary disease, thrombosis, chronic inflammatory disease, diabetes, ischemia, rheumatoid arthritis, cardiac infarct, cancer, hypertension, ocular damage, ischemia-reperfusion injury, and septic shock. In addition, the antioxidant heparin-nitroxide derivative of the invention may also be used for the preservation of biological transplants.

A further aspect of the invention concerns the use of the antioxidant heparin-nitroxide derivative of the invention as a contrast agent for MRI and as an EPR active redox-, pH-, or oxymetry- probes in EPRI. Potentially, the antioxidant heparin-nitroxide derivative of the invention is suitable monitoring local vascular oxidative stress by means of EPRI approach.

Accordingly, the invention comprises in a further aspect a method for electron paramagnetic resonance (EPR) imaging of vascular structure, comprising:
(a) Contacting vascular tissue with an paramagnetic heparin-nitroxide derivative of the invention,
(b) Washing of unbound heparin-nitroxide derivatives,
(c) Measuring of EPR signals obtained from vascular-bound paramagnetic heparin-nitroxide derivatives.

The inventors found that the heparin-nitroxide in solution exhibits an EPR signal that is typical for nitroxyl radicals. The signal is slightly broader than the EPR signal of TEMPOL. In addition, heparin-nitroxide binds to vascular tissue and can not be washed out by Krebs solution. However, it can be replaced by conventional heparin, indicating the competition for the same binding site.

For example, rat aorta contains about 10⁶-10⁷ binding sites per cell that can be efficiently occupied by the antioxidant heparin-nitroxide derivatives of the invention. As further shown by the inventors, the antioxidant activity of the heparin-nitroxide derivate of the invention is comparable to the cyclic nitroxide TEMPOL. At low concentrations, heparin-nitroxide effectively prevents the binding of myeloperoxidase (the most devastating free radical generating enzyme) to human umbilical vein endothelial cells (HUVECs).

The antioxidant and paramagnetic properties of the antioxidant heparin-nitroxide derivative (in short: H-NR) of the invention will be more apparent in the light of the following experiments, the results of which are shown in the accompanying Figures.

### Figure Legends

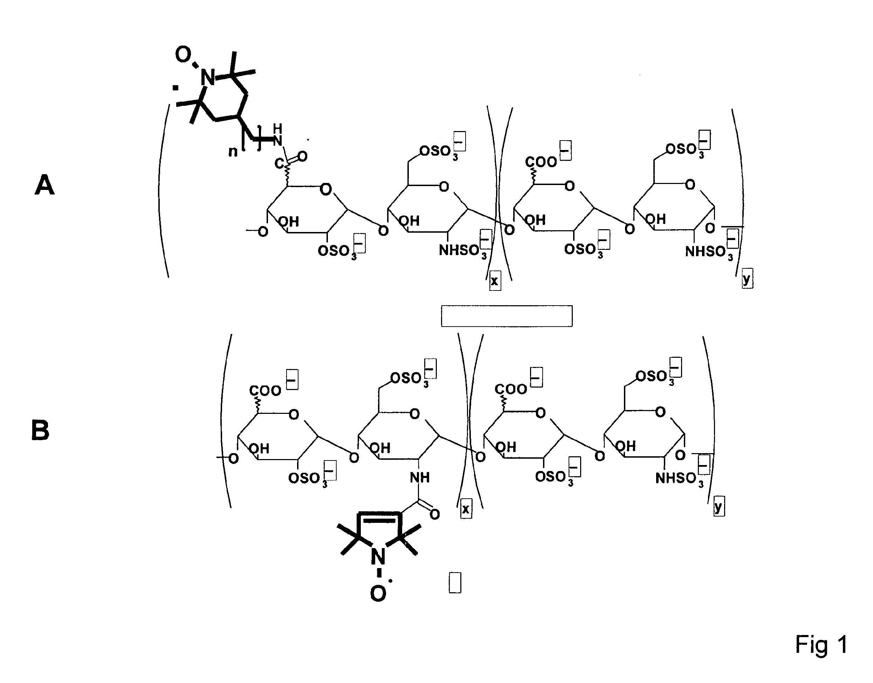

**Figure 1****.** General structure of the antioxidant heparin-nitroxides derivatives of the invention. There are two principal ways for conjugation of heparin with cyclic nitroxides that are preferred in the present invention: derivatization of heparin via carboxyl groups (A) and derivatization of heparin via amino groups (after N-desulfation) (B).
   The biological properties and EPR characteristics were studied using the following antioxidant heparin-nitroxide (H-NR) preparations:
   **H-NR-1:** Nitroxide conjugated with heparin via carboxyl group without long linker (20% disaccharides modified by TEMPO, FW ∼18000).
   **H-NR-2:** Nitroxide conjugated with heparin via carboxyl group without long linker (72% disaccharides modified by TEMPO, Few ∼18000).
   **H-NR-3:** Nitroxide conjugated with heparin via carboxyl group with a long linker (Hep-C(O)HN(CH₂)₅C(O)NH-R¹) (45% disaccharides modified by TEMPO, FW ∼18000).
   **H-NR-4:** Heparin conjugation with nitroxide via amino group without long linker (65% disaccharides modified by TEMPO, FW ∼18000).
   H and Hep, respectively, designate heparin. In Hep-C(O)NH-R¹ the group C(O)NH functions as a linker. FW designates the molecular weight of the whole construct, i.e. H-NR. Preferably, heparin is used with a molecular weight of around 15 KDa.

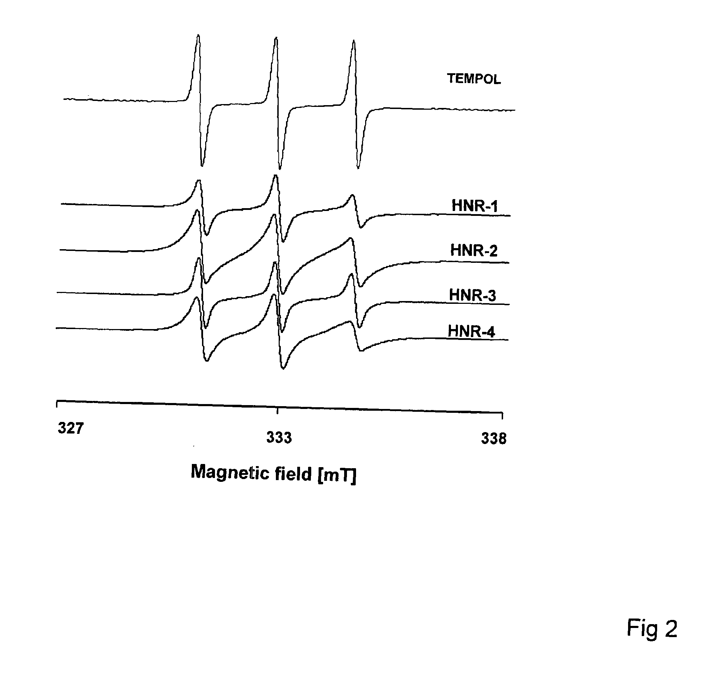

**Figure 2****.** Spectra EPR of TEMPO and different H-NRs (0.1 mM aqueous solutions, pH 7.4). EPR spectra were recorded at room temperature using an X-band radiospectrometer MS200 (Magnettech GmbH, Berlin). Instrument parameters were 10 mW microwave power, 0.1 mT amplitude modulation, 100 kHz modulation frequency, sweep field 11 mT and 120 s sweep time.
   In solution all four H-NRs exhibited triplet EPR signals (a_{N}∼17 gauss), which is typical for nitroxyl radicals. The line widths of the EPR signals of all H-NRs were slightly broader in comparison to the EPR signal of TEMPOL, indicating slight decrease in the mobility of nitroxyl groups upon binding to heparin as well as spin-spin interactions between them. Thus, ΔH (in gauss) were 1.8, 2.1, 2.5, 2.0 and 2.5 for TEMPOL, H-NR-1, H-NR-2, H-NR-3 and H-NR-4, respectively. The line width the signal is critical for the in vivo EPR imaging. A narrower signal provides for better resolution, and is therefore preferred. As a result, the H-NR-3, in which nitroxide is bound to heparin via a linker, provides for a higher spectral mobility, and is thus preferred for EPR imaging.

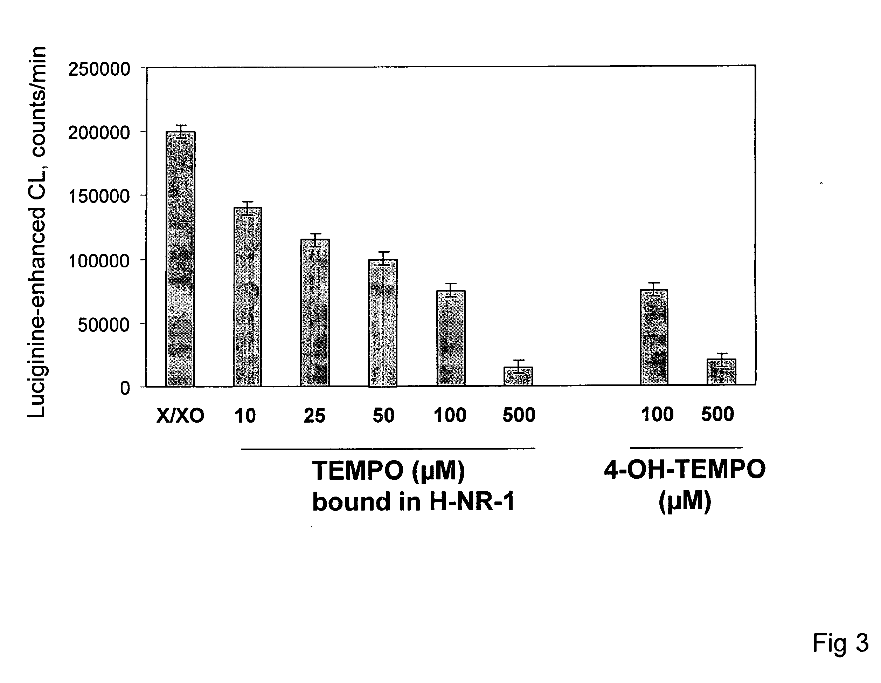

**Figure 3****.** Comparison of the superoxide scavenging properties of the heparin bound TEMPO groups and 4-hydroxy-TEMPO. Superoxide production was generated in the xantine (X, 0.5 mM) plus xantine oxidase (XO, 50 mU/ml) system (pH 7.4). The chemiluminescence signals were recorded in the presence of 50 µM lucigenin, 1 mM DTPA and a test compound using chemiluminometer Lumat 9507. The concentration of the heparin-bound TEMPO groups was calculated from the known content of the TEMPO-modified disaccharides (20 % in H-NR-1). Mean±SEM are shown for 4 different measurements.
   TEMPO is known to be an excellent antioxidant acting via scavenging of superoxide in a similar manner as superoxide dismutase. Thus, the inventors compared the superoxide scavenging activity of H-NR and TEMPO using the lucigenin-enchanced chemiluminescence assay and the superoxide generation system: xantine plus xantine oxidase.
   As shown in Fig 3, the superoxide scavenging activity of TEMPO groups-bound to heparin was comparable to the activity of 4-Hydroxy-TEMPO. Thus, in homogenous aqueous solution, the binding of TEMPO groups to heparin does not change its reactivity to superoxide. For the first time the inventors received a heparin derivative with antioxidant and paramagnetic properties.

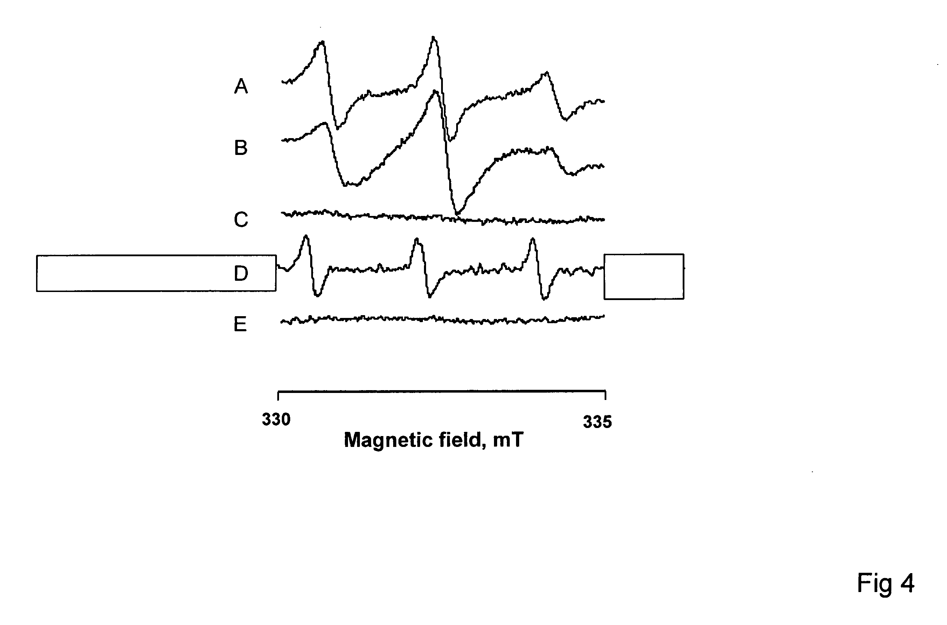

**Figure 4****.** Spectra EPR, (A) 10 µM H-NR-1 solution; (B) rat aortic ring (3 mm long) pre-incubated with 10 µM H-NR-1 for 1 hour and then washed 3 times with Krebs buffer; (C) rat aortic ring (3 mm long) pre-incubated with 10 µM H-NR-1 in the presence of heparin (liquemin, 30 units/ml) for 1 hour and then washed 3 times with Krebs buffer; (D) 5 µM 4-hydroxy-TEMPO; (E) rat aortic ring (3 mm long) pre-incubated with 10 µM 4-hydroxy-TEMPO for 1 hour and then washed 3 times with Krebs buffer. EPR spectra were recorded at room temperature using an X-band radiospectrometer MS200 (Magnettech GmbH, Berlin). Instrument parameters were 10 mW microwave power, 0.1 mT amplitude modulation, 100 kHz modulation frequency, sweep field 5 mT and 60 s sweep time. Representative spectra of 3 experiments are shown.
   The modification of heparin by nitroxide (especially via carboxyl group) may change the biological properties of heparin, i.e. its affinity to the heparan sulphate proteoglycans of the vascular extracellular matrix. The experiments have unequivocally shown that H-NR-1sticks to rat aorta with high affinity and can not be washed out thereafter with Krebs solution (Fig 4B). The fact that the H-NR-1 EPR signal was absent in aorta but in the presence of excess of heparin (Fig 4 C) indicates that H-NR-1 and non-modified heparin compete for the same binding sites. In contrast, incubation of aortas with 4-hydroxy-TEMPO did not result in the appearance of the EPR signal in tissue (Fig 4D).
   According to the inventor's calculations, rat aorta contains about 10⁶-10⁷ binding sites per cell. The half-life of the heparin-nitroxide in isolated vascular tissue is several hours at 37°C. These results demonstrate that the novel, EPR visible, heparin with antioxidant properties, can be efficiently targeted to vascular tissue and remains bound at extracellular sites.

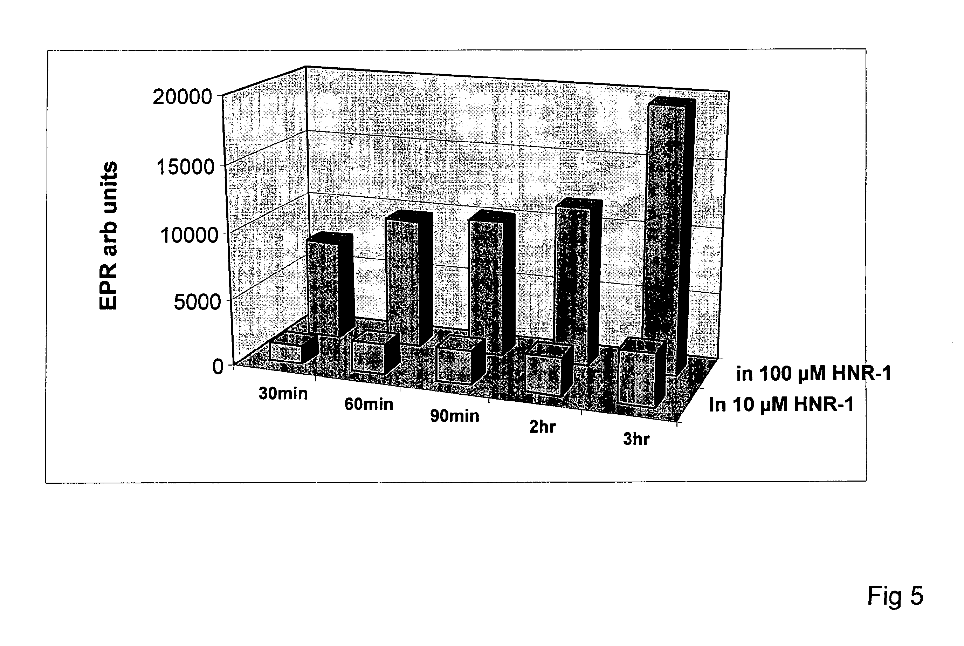

**Figure 5****.** The binding of H-NR-1 to rat aorta as function of time and concentration. Rat aortic rings (3 mm long) were incubated (37°C) with 10 µM or 100 µM H-NR-1 either for 0.5 hr, 1 hr, 1.5 hr, 2 hr, 3 hr and then washed out with Krebs solution. To estimate the amount of H-NR-1 bound to tissue, aortic rings were placed in capillaries, and spectra EPR (X-band) were recorded.
   The results demonstrate the concentration and time dependent accumulation of H-NR in rat aorta, and reflect the rate of penetration of H-NR into the vascular wall.

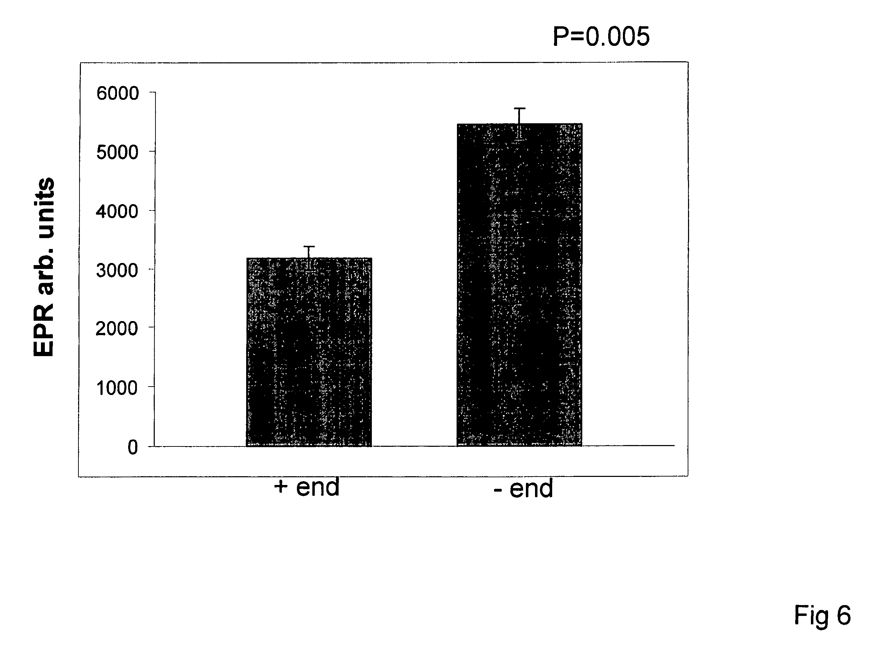

**Figure 6****.** Enhanced binding of H-NR in the endothelium-denuded rat aorta. Rat aortic rings (3 mm long) with or without endothelium were incubated (37°C) with 10 µM H-NR-1 for 1 hr and then washed out with Krebs solution. To estimate the amount of H-NR-1 bound to tissue, aortic rings were placed in capillaries and spectra EPR (X-band) were recorded at room temperature.
   The results obtained in these experiments suggest that the endothelium acts as a barrier for H-NR-1 penetration into the vascular wall. The large macromolecule heparin is known to have affinity not only to the endothelial cell surface but also to a variety of vascular extracellular matrix proteins including fibronectin and collagen. Vascular pathology is known to be associated with the endothelium insufficiency and remodelling of extracellular matrix. Potentially H-NR-1 can be preferably accumulated in the problematical sites of the vascular wall (i.e. early stages of atherosclerotic plaques) and by this way provides the local antioxidant defence in strategically important site. On the other hand, the EPR detection of the disturbed signal parameters/kinetic in a problematic zone, potentially can be used for a diagnostic purpose.

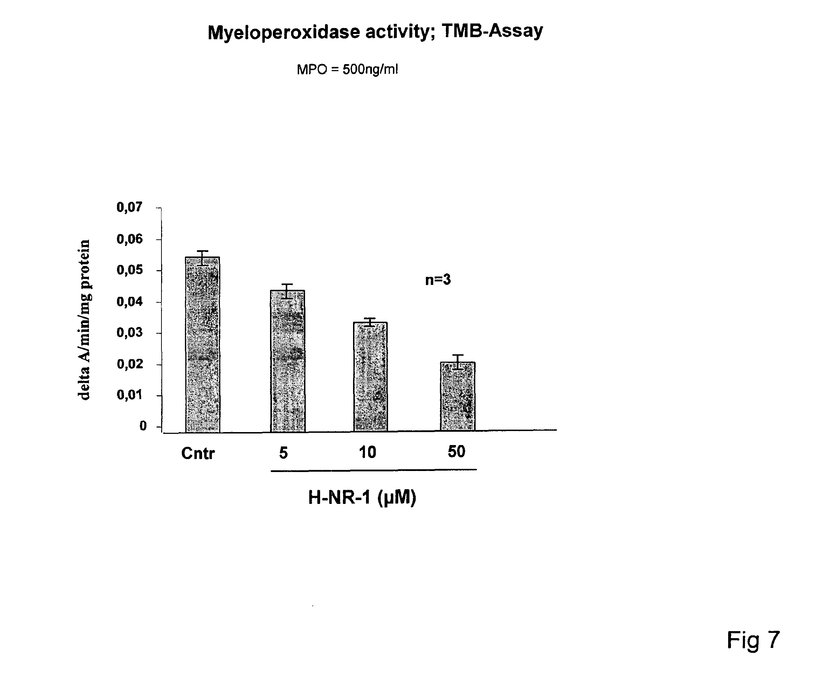

**Figure 7****.** Myeloperoxidase (MPO, Sigma; 500 ng/ml) activity was measured in the presence of increasing concentrations of H-NR-1 using the 3,3',5,5'-tetramethylbenzidine (TMB) assay kit (Calbiochem). The absorbance of the oxidized TMB was detected at 450 nm. Measurements were performed in the laboratory of Dr. S. Baldus (University Hospital Hamburg-Eppendorf).
   MPO has emerged as a critical mediator of inflammatory vascular diseases, such as atherosclerosis. MPO has been show to be accumulated in atherosclerotic plaques where it can oxidise high-density lipoproteins, activate metalloproteinases and exert cytokine-like property. Our results indicate that H-NR can directly inhibit the MPO activity (presumably via scavenging of MPO derived oxidants) and thus H-NR may become an effective drug against the myeloperoxidase-dependent vascular tissue damage.

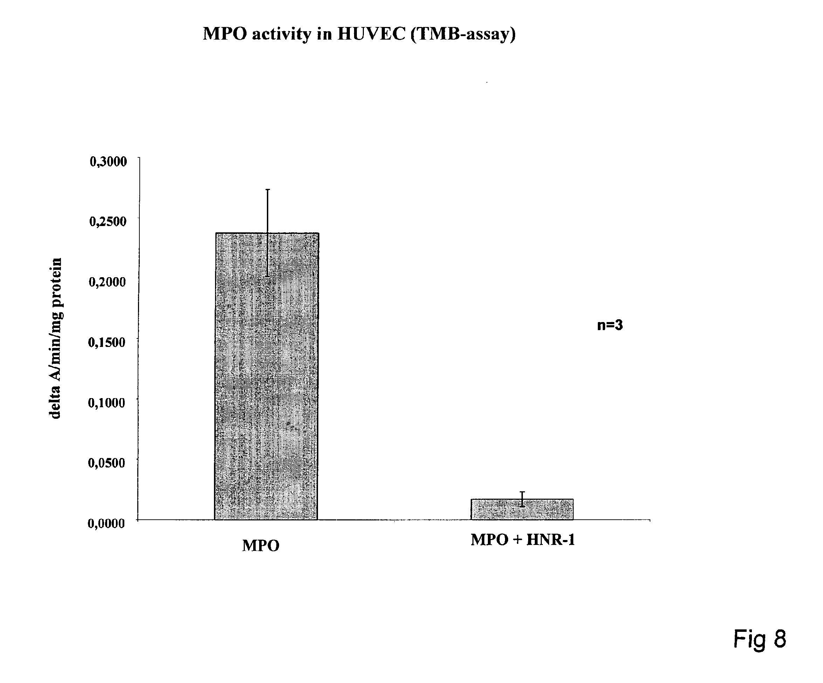

**Figure 8****.** Effect of H-NR-1 on the myeloperoxidase (MPO) activity (TMB assay) in human umbilical vein endothelial cells (HUVEC) lysate. HUVEC were pre-incubated with myeloperoxidase (MPO; 1 µg/ml) for 1 hr; then non-bound MPO was washed out and cells were further incubated in the presence or absence of H-NR-1 (50 µM) for additional 30 min. After the incubation cells were washed out, lysed and MPO activity was measured using the TMB assay kit (Calbiochem). Measurements were performed in the laboratory of Dr. S. Baldus (University Hospital Hamburg-Eppendorf)
   These results indicate that H-NR can struggle the MPO-dependent deleterious effects in human endothelial cells.

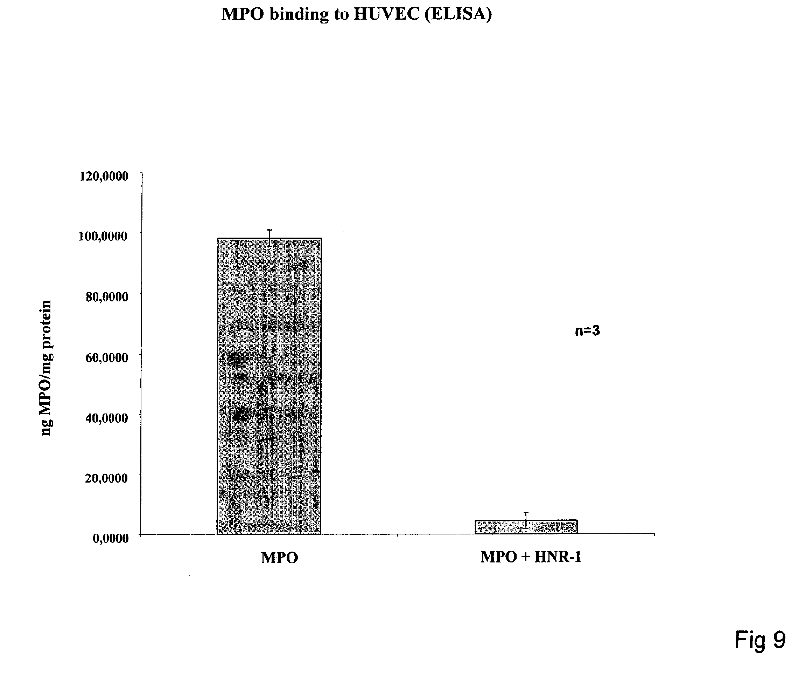

**Figure 9****.** Effect of H-NR-1 on the binding of myeloperoxidase (MPO) to human umbilical vein endothelial cells (HUVEC). HUVEC were pre-incubated with myeloperoxidase (MPO; 1µg/ml) for 1 hr; then non-bound MPO was washed out and cells were further incubated in the presence or absence of H-NR-1 (50 µM) for additional 30 min. After the incubation period, cells were washed out, lysed and MPO-protein content was determined by ELISA (Calbiochem). Measurements were performed in the laboratory of Dr. S. Baldus (University Hospital Hamburg-Eppendorf)
   These results indicate that H-NR not only can directly scavenge the MPO-derived oxidants, but also replace the bound MPO from endothelium.

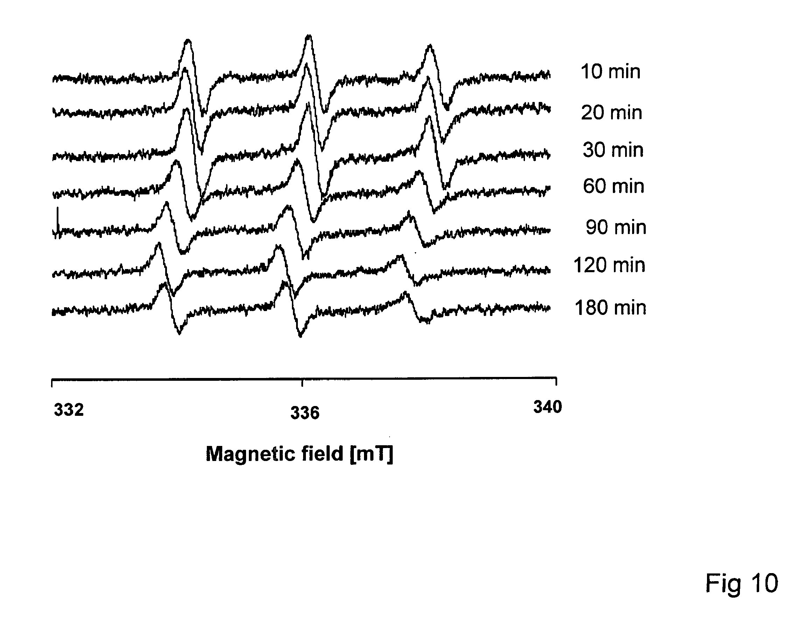

**Figure 10****.** In vivo EPR evidence for prolonged life time of H-NR. Anesthetized mice were injected with 1 mM H-NR-3 (0.5 ml-i.p.). The mouse tail was fixed in the resonator of a X-band EPR spectrometer (MS 200 Magnettech) and the spectra EPR were sequentially recorded.
   It is likely that both H-NR circulating with blood and H-NR bound to vascular wall are responsible for these EPR spectra. Most importantly, the half-life of TEMPOL in vivo is known to last only a few minutes, while the half-life of H-NR according to the invention in vivo lasts several hours. These results substantiate the potential utilization of H-NR as perspective agents for in vivo EPR imaging.

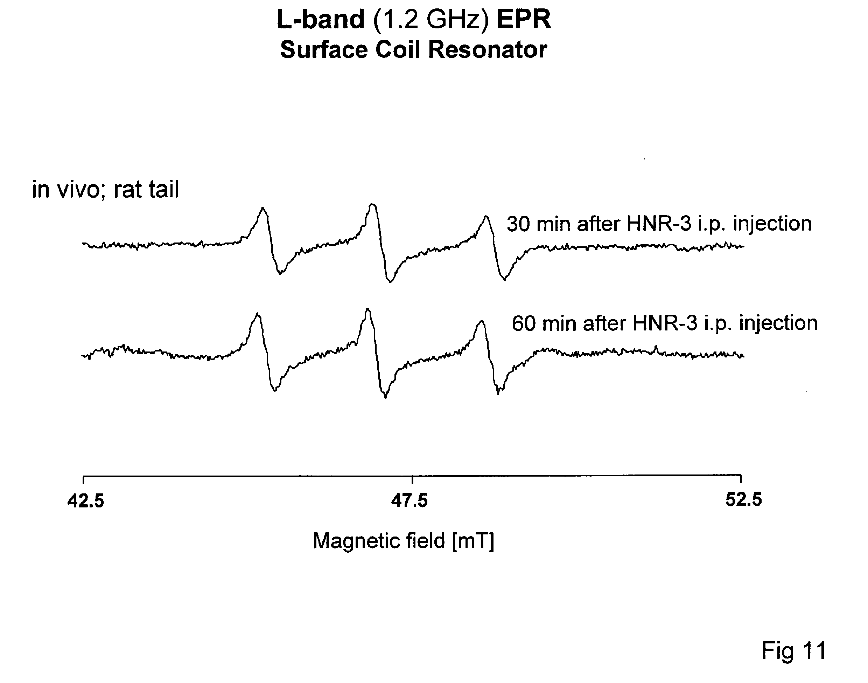

**Figure 11****.** EPR (L-band) evidence for the prolonged life-time of H-NR in anesthetized mice (i.p. injection of 0.5 ml-1mM solution of H-NR-3). The surface coil-type resonator (loop diameter 10 mm) was placed on the proximal part of mouse tail and the spectra EPR were recorded using an L-band (1.2 GHz) spectrometer RadicalScope mt 500L (Magnettech GmbH, Berlin). Instrument parameters were 25 mW microwave power, 0.125 mT amplitude modulation, 100 kHz modulation frequency, center field 47.5 mT, sweep field 10 mT and 60s sweep time.
   This experiments shows that H-NR may be efficiently used as imaging agent in vivo. As such it serves as an potential contrasting agent for EPR imaging of vascular structures. In addition, the H-NR of the invention may be used for treating vascular diseases and for the preservation of vascular transplants.

## Claims

1. Antioxidant heparin-nitroxide derivative, comprising heparin or a derivative thereof, and one or more nitroxides or derivatives thereof, wherein the nitroxide or derivative thereof is covalently coupled to heparin or a derivative thereof by derivatization of glycosaminoglycan reactive groups.

2. Antioxidant heparin-nitroxide derivative of claim 1, wherein the nitroxide or derivative thereof is coupled to the heparin macromolecule or derivative thereof by derivatisation of glycosaminoglycan carboxyl groups and/or glycosaminoglycan amino groups.

3. Antioxidant heparin-nitroxide derivative of claim 1 or claim 2, wherein the antioxidant heparin-nitroxide derivative comprises the following general structure: 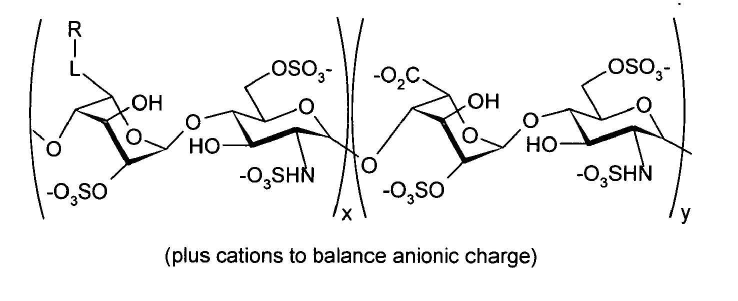 or 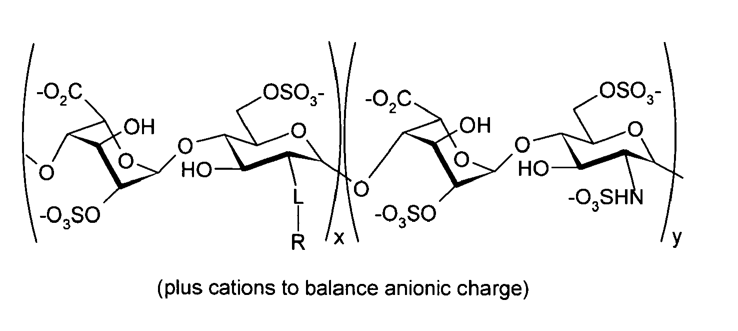 wherein the nitroxide R is a 5- or 6-atom N-heterocycle, L is a linker which comprises amide -C(O)NR'-, ester -C(O)O- or their combination separated by hydrocarbon chain -C(O)NR'-(CH₂)ₙ₋C(O)O- or by any other suitable linker, R' is a hydrogen or an alkyl substituent, n indicates the chain length of the linker, wherein n ≥ 0, and x,y > 0.

4. Antioxidant heparin-nitroxide derivative of any of the preceding claims, wherein the nitroxide is coupled to heparin via a linker L comprising the general structure (CH₂)ₙ, wherein n > 0.

5. Antioxidant heparin-nitroxide derivative of any of the preceding claims, wherein the nitroxide or derivative thereof is derived from cyclic nitroxides.

6. Antioxidant heparin-nitroxide derivative of any of the preceding claims, wherein the nitroxide or derivative thereof is derived from piperidine, tetrahydropyridine, pyrroline, pyrrolidine, imidazoline, imidazolidine or oxazolidine.

7. Antioxidant heparin-nitroxide derivative of any of the preceding claims, wherein the nitroxide is TEMPO (2,2,6,6-tetramethylpiperidine-1-oxyl).

8. Antioxidant heparin-nitroxide derivative of any of the preceding claims, wherein the derivatization of carboxylate groups in heparin is defined by one of the following structures of groups (I) - (IV):
(I) 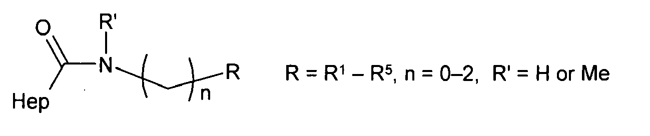
(II) 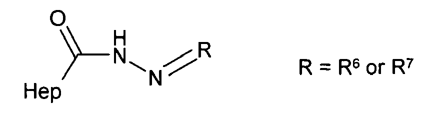
(III) 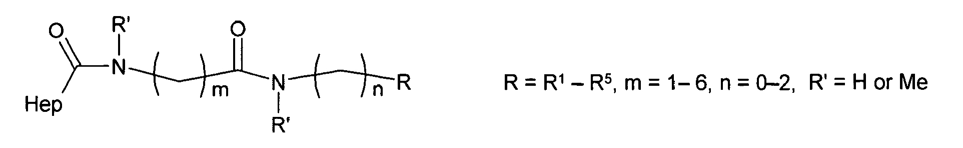 or 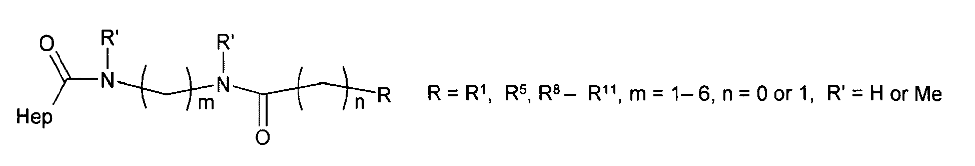
(IV) 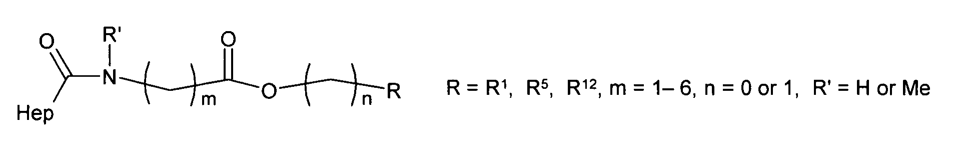 or 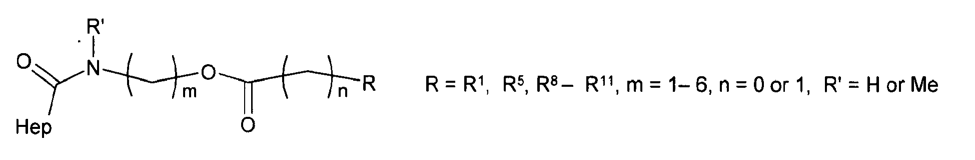 wherein R = R¹⁻¹² may be one of the following residues:
R¹ = 2,2,6,6-tetramethyl-1-oxylpiperidin-4-yl,
R² = 3-amino-2,2,6,6-tetramethyl-1-oxylpiperidin-4-yl,
R³ = 4-alkyloxycarbonyl-2,2,6,6-tetramethyl-1-oxylpiperidin-4-yl,
R⁴ = 4-hydroxyimino-2,2,6,6-tetramethyl-1-oxylpiperidin-3-yl,
R⁵ = 2,2,5,5-tetramethyl-1-oxylpyrrolidin-3-yl,
R⁶ = 2,2,6,6-tetramethyl-1-oxylpiperidin-4-diyl,
R⁷ = 2,2,5,5-tetramethyl-1-oxylpyrrolidin-3-diyl,
R⁸ = 2,2,6,6-tetramethyl-1-oxyl-1,2,5,6-tetrahydropyridin-4-yl,
R⁹ = 4-acetylamino-2,2,6,6-tetramethyl-1-oxylpiperidin-4-yl,
R¹⁰ = 2,2,5,5-tetramethyl-1-oxylpyrrolin-3-yl,
R¹¹ = 2,2,5,5-tetramethyl-4-bromo-1-oxylpyrrolin-3-yl,
R¹² = 2,2,6,6-tetramethyl-1-oxyl-4-phenylpiperidin-4-yl.

9. Antioxidant heparin-nitroxide derivative of any of the preceding claims, wherein the derivatization of carboxylate groups in heparin is defined by one of the following structures of groups (I) - (III):
(I) 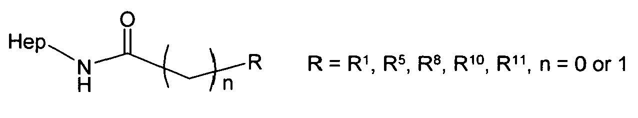
(II) 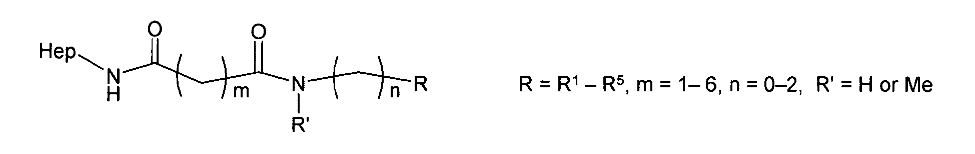 or 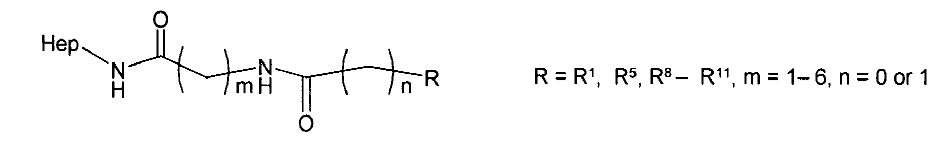
(III) 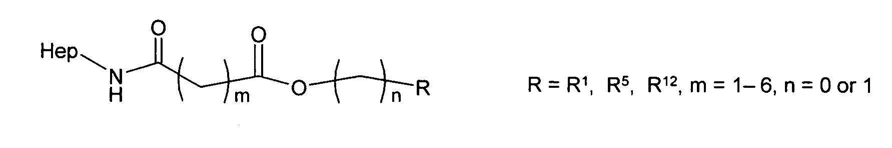 wherein R = R¹⁻¹² may be one of the following residues:
R¹ = 2,2,6,6-tetramethyl-1-oxylpiperidin-4-yl,
R² = 3-amino-2,2,6,6-tetramethyl-1-oxylpiperidin-4-yl,
R³ = 4-alkyloxycarbonyl-2,2,6,6-tetramethyl-1-oxylpiperidin-4-yl,
R⁴ = 4-hydroxyimino-2,2,6,6-tetramethyl-1-oxylpiperidin-3-yl,
R⁵ = 2,2,5,5-tetramethyl-1-oxylpyrrolidin-3-yl,
R⁶ = 2,2,6,6-tetramethyl-1-oxylpiperidin-4-diyl,
R⁷ = 2,2,5,5-tetramethyl-1-oxylpyrrolidin-3-diyl,
R⁸ = 2,2,6,6-tetramethyl-1-oxyl-1,2,5,6-tetrahydropyridin-4-yl,
R⁹ = 4-acetylamino-2,2,6,6-tetramethyl-1-oxylpiperidin-4-yl,
R¹⁰ = 2,2,5,5-tetramethyl-1-oxylpyrrolin-3-yl,
R¹¹ = 2,2,5,5-tetramethyl-4-bromo-1-oxylpyrrolin-3-yl,
R¹² = 2,2,6,6-tetramethyl-1-oxyl-4-phenylpiperidin-4-yl.

10. Antioxidant heparin-nitroxide derivative of any of the preceding claims, wherein about 20 % to 70 % of the disaccharides of heparin are modified by TEMPO (2,2,6,6-tetramethylpiperidine-1-oxyl).

11. Antioxidant heparin-nitroxide derivative of any of the preceding claims, wherein around 20 % of the disaccharides of heparin are modified by TEMPO (2,2,6,6-tetramethylpiperidine-1-oxyl).

12. Antioxidant heparin-nitroxide derivative of any of the preceding claims, wherein around 45 % of the disaccharides of heparin are modified by TEMPO (2,2,6,6-tetramethylpiperidine-1-oxyl).

13. Antioxidant heparin-nitroxide derivative of any of the preceding claims, wherein around 70 % of the disaccharides of heparin are modified by TEMPO (2,2,6,6-tetramethylpiperidine-1-oxyl).

14. Antioxidant heparin-nitroxide derivative of any of the preceding claims, wherein heparin or a derivative thereof has a molecular weight of approximately between 5 and 40 kDa.

15. Antioxidant heparin-nitroxide derivative of any of the preceding claims, wherein heparin or a derivative thereof has a molecular weight of 15 kDa.

16. Method for production of antioxidant heparin-nitroxide derivatives, comprising the step of:
(a) Derivatization of glycosaminoglycan reactive groups with nitroxide or a derivative thereof.

17. Method of claim 16, wherein the derivatization is performed on nitroxide amino, carboxyl, carboxylic acid anhydride or isocyano groups.

18. Pharmaceutical composition, comprising an antioxidant heparin-nitroxide derivative of any one of claims 1 to 15.

19. Use of an antioxidant heparin-nitroxide derivative of any one of claims 1 to 15 for the preparation of a medicament for treatment of acute and chronic diseases associated with oxidative extracellular stress.

20. Use of claim 19, wherein the disease is selected from the group consisting of oxidative stress-dependent platelet activation, cardiovascular disease, neurodegenerative diseases such as Alzheimer's, pulmonary disease, thrombosis, chronic inflammatory disease, diabetes, ischemia, rheumatoid arthritis, cardiac infarct, cancer, hypertension, ocular damage, ischemia-reperfusion injury, and septic shock.

21. Agent for electron paramagnetic resonance imaging (EPRI) and for magnetic resonance imaging (MRI) comprising an antioxidant heparin-nitroxide derivative of any one of claims 1 to 15.

22. Use of an antioxidant heparin-nitroxide derivative of any one of claims 1 to 15 for electron paramagnetic resonance imaging (EPRI) of intimal layer of blood vessels.

23. Use of an antioxidant heparin-nitroxide derivative of any one of claims 1 to 15 for monitoring local vascular oxidative stress, pH values or oxygen levels.

24. Use of an antioxidant heparin-nitroxide derivative of any one of claims 1 to 15 for the preservation of biological transplants.

25. Method for electron paramagnetic resonance imaging (EPRI) of vascular structure, comprising:
(a) Contacting vascular tissue with a paramagnetic heparin-nitroxide derivative of any one of claims 1 to 15,
(b) Washing of unbound heparin-nitroxide derivatives,
(c) Measuring of EPR signals obtained from vascular-bound paramagnetic heparin-nitroxide derivatives.
